# EUROPEAN PATENT APPLICATION

(11) **EP 4 321 159 A1**
(43) Date of publication of application: **14.02.2024**
(21) Application number: 23190409.5
(22) Date of filing: 08.08.2023
(51) Int. Cl.: A61K 31/42, A61K 31/4375, A61K 31/495, A61P 17/14

(54) **NOVEL USE OF GPR91 INHIBITORS**

(30) Priority: 09.08.2022 US 202263396365 P
(71) Applicant: GlaxoSmithKline Intellectual Property Development Limited, Stevenage SG1 2NY (GB)
(72) Inventor: AIRA DIAZ, Lazaro, Collegeville, 19426 (US); PANIGEL, Jacqueline, Collegeville, 19426 (US)
(74) Representative: Matley, Joshua Ellis

(57) **Abstract**

The present disclosure relates to an inhibitor of GPR91 for use in the treatment or prevention of hair-loss related disorders such as androgenetic alopecia. Combination therapies are also described. The present disclosure also provides an inhibitor of GPR91 for use in inducing or promoting hair growth in a human and methods thereof.

## Description

### FIELD OF THE DISCLOSURE

The present disclosure relates to an inhibitor of GPR91 for use in the treatment or prevention of hair-loss related disorders such as androgenetic alopecia. Combination therapies are also described.

### BACKGROUND TO THE DISCLOSURE

Body Hair grows in cycles consisting of three phases: anagen (long growing phase), catagen (brief transitional apoptotic phase) and telogen (a short resting phase). Hair falls out at the end of the telogen phase (exogen phase), allowing the cycle to begin again and for a new hair to start growing in the follicle. Hair-loss may occur when a significant number of hairs reach the end of the resting phase (typically more than 100 hairs a day) resulting in clinical hair loss (telogen effluvium). A disruption of the growing phase causing abnormal loss of anagen hairs is an anagen effluvium. Forty percent of males demonstrate noticeable hair loss by age 35, 65% by age 60, and 80% by age 80. Alopecia affects approximately 50% of females. The number of hair loss sufferers, world-wide, seeking professional treatment has more than doubled in the last decade. This hair loss is typically caused by alopecia areata, alopecia universalis, alopecia totalis, androgenetic alopecia, or telogen effluvium.

Alopecia Areata (AA) is one of the most highly prevalent autoimmune diseases, leading to hair loss due to the collapse of immune privilege of the hair follicle and subsequent autoimmune destruction. AA is a skin disease which leads to hair loss on the scalp and elsewhere.

Androgenetic alopecia (AGA) is a hereditary, androgen induced, age dependent loss of hair on the scalp. It is the most common cause of alopecia and accounts for up to 50% of hair loss experienced by males and females. In both men and women, AGA is characterized by a distinctive patterned loss of hair. In males, hair loss typically occurs most prominently in the vertex and frontotemporal regions of the scalp, and is commonly known as male pattern baldness as hair loss is in a defined pattern. In women, hair loss is typically more diffuse at the crown and top of the hair, particularly at the hairline part.

The pathogenesis of AGA includes alteration to the dynamics of the hair follicle cycle as well as progressive miniaturization of the hair follicle itself. This miniaturization results in the conversion of terminal hairs into secondary vellus hairs which can be recognized in scalp biopsies. Changes in the hair follicle cycle in AGA affected hair follicles involve a shortening of the anagen (growth) phase and increased length of telogen (resting) phase.

The known molecular drivers involved in AGA pathogenesis include overactive activity of the androgen receptor (AR). It is well established that androgen involvement is required for the development of AGA. Men who are deprived of testicular androgens through castration before puberty do not develop AGA. On the other hand, there are known genetic predispositions that increase levels of the type II 5alpha-reductase enzyme, which increases the conversion of testosterone to dihydrotestosterone (DHT), a much more potent agonist of AR leading to androgen dependent hair loss.

Limited therapeutic interventions have been directed specifically at modulating androgen metabolism. Finasteride is an FDA approved therapy that targets the 5-alpha reductase enzyme. This medication has limited success in the clinic and has significant adverse side effects associated with long term use. Dutasteride is approved in Korea and Japan and inhibits both type I and type II 5-alpha reductase. While AR signalling is required for the development of AGA, it is not sufficient to account for all of the pathophysiological hair follicle disturbances in AGA.

Therefore, there is a significant need to identify and develop new treatments for hair-loss related disorders.

### SUMMARY OF THE DISCLOSURE

One aspect of the disclosure is an inhibitor of GPR91 for use in the treatment or prevention of hair-loss related disorders or a symptom thereof in a subject in need thereof.

Another aspect of the disclosure is a method for treating or preventing hair-loss related disorders or a symptom thereof in a subject in need thereof comprising administering to a human a therapeutically effective amount of an inhibitor of human GPR91.

Another aspect of the disclosure is an inhibitor of GPR91 for use in the manufacture of a medicament for use in the treatment or prevention of a hair-loss related disorders or a symptom thereof.

Another aspect of the disclosure is an inhibitor of GPR91 for use in inducing or promoting hair growth in a human.

Another aspect of the disclosure is a method of inducing or promoting hair growth in a human, the method comprising administering an effective amount of an inhibitor of GPR91.

### DESCRIPTION OF DRAWINGS/FIGURES

Figures 1A, 1B and 1C demonstrate that hair follicle relevant immune cells (mast cells, macrophages) have GPR91 mRNA expression and that in macrophages, the M2 phenotype expresses the highest levels of GPR91.
Figure 1D demonstrates that GPR91 is not expressed in hair follicle cells such as the dermal papilla and hair follicle keratinocytes.
Figures 1E and 1F demonstrate that hair follicle relevant immune cells (mast cells and macrophages) express GPR91 protein at the cell membrane.
Figures 2A and 2B demonstrate that a cytokine response in LUVA cells treated with succinate for 3 or 72 hours is GPR91 dependent which is evidenced by the lack of cytokine secretion in the GPR91 knockout cells.
Figure 3A demonstrates that skin resident human mast cells and macrophages in a mouse model engrafted with human CD34+ hematopoietic stem cells express GPR91 protein at the cell membrane.
Figure 3B demonstrates that GPR91 is expressed in both healthy and AGA human hair follicles in cells residing in the connective tissue sheath that morphologically resemble immune cells. GPR91 positive expression may be observed in perifollicular mast cells and macrophages by co-staining with macrophage (CD68 or other) and mast cell (cKIT or other) markers.
Figure 4 demonstrates that the presence of succinate during macrophage polarization leads to higher levels of expression of TREM2 in human iPSC derived M2 macrophages.
Figure 5 demonstrates that disease relevant TREM2 expressing human iPSC derived M2 macrophages secrete higher levels of oncostatin M (a cytokine shown to induce hair follicle quiescence in mice) after treatment with succinate for 48 hr.
Figures 6 demonstrates that succinate induced oncostatin M secreted by TREM2 expressing M2 macrophages can be reduced by the co-administration of a GPR91 inhibitor. Figure 6A and Figure 6B represent the same experimental paradigm from two independent iPSC donors.
Figures 7A and 7B demonstrate that secreted factors in conditioned media isolated from iPSC derived macrophages treated with succinate inhibit dermal papilla proliferation (from both male and female donors), and that the action of these succinate-mediated deleterious secreted factors in dermal papilla cells can be prevented by the co-administration of a GPR91 inhibitor.
Figure 7C demonstrates that succinate by itself does not induce any changes in the proliferation of dermal papilla cells suggesting that its effect on proliferation occurs indirectly through a paracrine signaling mechanism from perifollicular immune cells.
Figure 8 demonstrates that recombinant oncostatin M applied to dermal papilla activates the JAK-STAT signaling pathway, as evidenced by the phosphorylation of STATS and STATS which can be blocked by a JAK inhibitor (Tofacitinib)
Figure 9A and 9B demonstrate that recombinant oncostatin M applied to dermal papilla cells increases the expression of FGF 18 (which arrests the hair follicle cycle and maintains hair follicles in non-growing state) and downregulates expression of BMP6 (which is required for the maintenance of dermal papilla inductivity). The dashed line at 1 represents the control response.
Figure 10 demonstrates that oncostatin M induces senescence in the dermal papilla as measured by increased β-galactosidase activity.
Figures 11A and 11B demonstrates that GPR91 inhibitors increase the percentage of hair follicles in the anagen phase and prevent catagen in hair follicles from healthy/ non alopecia affected donors.
Figure 11C demonstrates the GPR91 inhibition in terminal and intermediate hair follicles from balding regions of the scalp of AGA affected donors increases the percentage of hair follicles in the anagen phase and prevents catagen.
Figure 11D demonstrates that GPR91 activation by succinate increases the percentage of hair follicles in the catagen phase.
Figures 12A and 12B are representative images of Fontana-Masson stained hair follicles demonstrating the increased size of the dermal papilla area observed with 2 concentrations (SuM and 50uM) of Compound 1 and the decreased size of the dermal papilla area as well as melanin clumping (a marker of toxicity) induced by succinate, a GPR91 activator.
Figure 13A demonstrates that Compound 1 increases the dermal papilla area in healthy hair follicles.
Figure 13B shows that GPR91 activation reduces the area of the dermal papilla in healthy hair follicles.
Figure 13C demonstrates that GPR91 inhibition by Compound 1 increases the dermal papilla area in AGA hair follicles, specifically in the affected/balding region of the scalp.
Figures 14A and 14B demonstrate that versican expression in the dermal papilla can be increased by the administration of a GPR91 inhibitor.

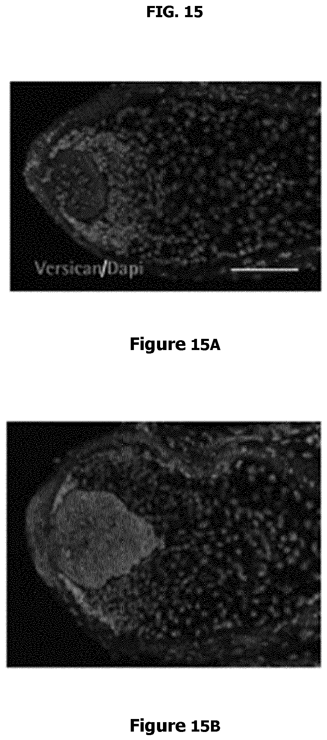

Figures 15A (control) and 15B (Compound 1) are immunofluorescent stains showing versican expression in the dermal papilla of healthy hair follicles. Increased versican expression is observed when a GPR91 inhibitor is administered.
Figures 16A and 16B demonstrate that succinate administration may reduce hair matrix keratinocyte proliferation and increases apoptosis in hair follicles from healthy donors.
Figures 17A to 17C demonstrate that GPR91 inhibition may positively regulate K15 stem cell proliferation in the bulge basal layer in hair follicles from healthy donors.
Figures 18A to 18C demonstrate that GPR91 activation may negatively regulate K15 stem cell proliferation in the bulge basal layer in hair follicles from healthy donors.
Figure 19A is a volcano plot that highlights the differentially expressed genes in ex vivo cultured hair follicles from healthy donors 24 hours after treatment with 3µM succinate. Genes that are considered significantly changed have a p value of <0.05 and have a fold change (FC) either greater than 2 (upregulated) or less than 0.5 (downregulated). This figure demonstrated that 28 genes were significantly upregulated, and 44 genes were significantly downregulated in response to succinate treatment.
Figure 19B highlights specific genes and the relevant biological processes they represent. After treatment with succinate, there is an increase in genes related to immune activation, specifically a cytokine and chemokine response. There is also a significant downregulation in genes that regulate hair follicle pathways as well as downregulations of keratins. These changes would be expected to coincide with increased perifollicular inflammation as well as decreased hair growth.

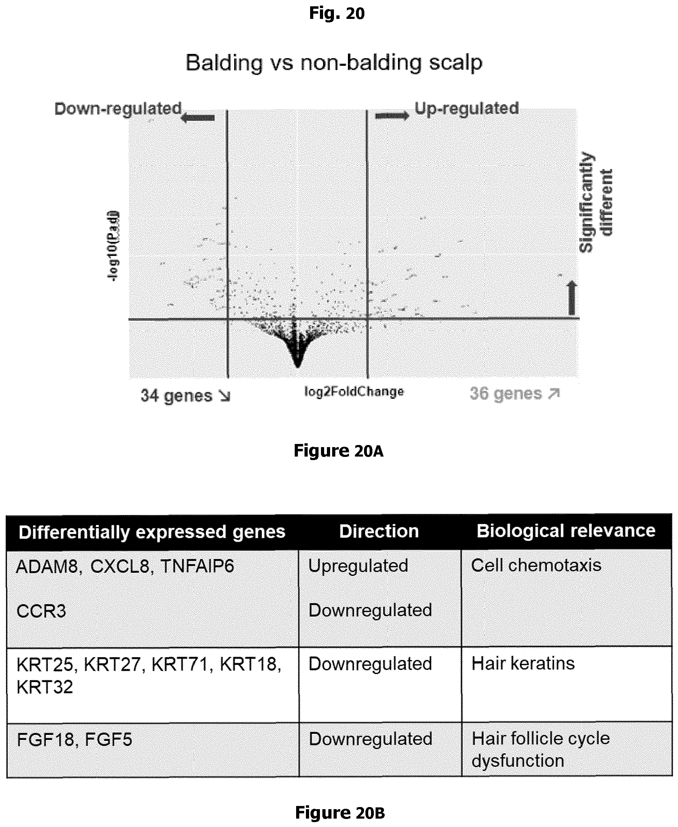

Figure 20A is a volcano plot that highlights differentially expressed genes in ex vivo cultured hair follicles from AGA patient donors. Hair follicles were isolated from both the affected region of the scalp (balding) as well as the non-affected region of the scalp (non-balding) to identify genes related specifically to balding. Genes that are considered significantly changed have a p value of <0.05 and have a fold change (FC) either greater than 2 (upregulated) or less than 0.5 (downregulated). This figure demonstrates that 36 genes were significantly upregulated, and 34 genes were significantly downregulated in balding hair follicles compared to non-balding hair follicles.
Figure 20B highlights specific genes and the relevant biological processes they represent. In balding scalp, there is an increase in genes related to cellular chemotaxis. There is also a significant downregulation in genes that regulate keratins and the hair follicle cycle. These changes would be expected to coincide with increased cell infiltration in balding scalp as well as decreased hair growth which is consistent with known AGA phenotypes.

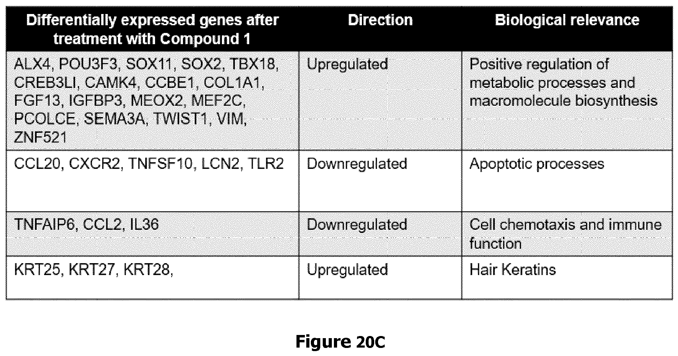

Figure 20C highlights specific genes and the relevant biological processes from differentially expressed genes that are observed after treatment of hair follicles from affected regions of the scalp with 5 nM Compound 1 for 24 hours. After treatment with Compound 1, there is an increase in genes related to increased metabolic processes and biosynthesis of macromolecules. There is also a significant downregulation in genes that play a role in apoptotic processes, cell chemotaxis and immune function. There is also a significant upregulation in hair keratins. These changes would be expected to coincide with reduced cellular trafficking in balding scalp, decreased apoptosis and increased hair growth which is consistent with mechanisms that are predicted to improve hair follicles affected by AGA.

### DETAILED DESCRIPTION OF THE EMBODIMENTS

### STATEMENT OF THE DISCLOSURE

Genome-wide association studies (GWAS) have been conducted for male pattern baldness cases and early onset hair loss (androgenetic alopecia).

Table 1 shows the genetic evidence associating GPR91 with AGA and highlights novel genetic data that identifies GPR91 as the causal gene at the locus identified in previous GWAS studies.

**TABLE 1**

| Traits | Index SNP_reference_ | Risk/Protective | P value | GPR91 Expression based on presence of risk or protective allele |
|---|---|---|---|---|
| Androgenetic Alopecia¹ | rs13060333_T_A | Risk: A | 2e-15 | Increase |
| Early onset of balding² | rs4679956_C_T | Risk: T | 2e-18 | Increase |
| UKB hair balding pattern | rs17283269_G_A | Risk: A | 6.2e-49 | Increase |
| UKB pattern 1 (not balding) | rs17283269_G_A | Protective: A | 6.7e-57 | Decrease |
| UKB pattern 3 | rs73168962_T_G | Risk G | 1.3e-16 | Increase |
| UKB pattern 4 | rs13060333_T_A | Risk: A | 1.9e-19 | Increase |
| 23andme self reported male hair loss | rs2723366_T_C rs4679956_C_T | Protective: C Risk:T | 3e-8 2.5e-68 | Decrease Increase |
| 23andMe age of balding onset | rs6772866_G_T | Protective: T (later onset) | 3.3e-13 | Decrease |
| 23andMe male pattern (age of onset <40) | rs145487317_T_31I | Risk: 31 bp insertion | 5.1e-20 | Increase |

This genetic analysis demonstrates that increased expression of GPR91 is associated with an early onset of balding and increased risk of balding whereas decreased GPR91 expression is associated with a protection from hair loss. The present disclosure demonstrates that inhibition of the GPR91 (also known as SUCNR1) receptor is capable of treating and/or preventing androgenetic alopecia.

The terms "treat", "treating", "treatment", or the like, as used herein mean to alleviate (reduce, minimise, or eliminate) symptoms, or to reduce, minimise or eliminate the causation of symptoms either on a temporary or permanent basis.

### GRP91

As used herein, "GPR91" refers to the G-protein coupled transmembrane receptor or succinate receptor encoded in humans by the nucleic acid (mRNA) sequence polypeptide with amino acid sequence encoded by SEQ ID No. 2 or an isoform, variant or ortholog of this sequence.

### GPR91 INHIBITOR

The GPR91 inhibitor may be any molecule that binds to the GPR91 receptor and modulates the biological activity. The precise inhibitor is not particularly limited and may be any molecule known in the art that has GPR91 inhibitory activity.

In one embodiment, the GPR91 inhibitor comprises a small molecule inhibitor, an antibody or fragment thereof, a binding protein, or an aptamer. The GPR91 inhibitor binds to the GPR91 receptor and modulates the biological activity.

In one embodiment, the GPR91 inhibitor is a small molecule. A small molecule is a compound that has a mass of less than 2000 Daltons. In one embodiment, the molecular mass is preferably less than 1000 Daltons. In one embodiment, the molecular mass is preferably less than 600 Daltons. For example, the molecular mass is less than 500 Daltons, less than 400 Daltons, less than 300 Daltons, less than 200 Daltons, or less than 100 Daltons.

The small molecule may be organic or inorganic provided that it inhibits the GPR91 receptor.

In one embodiment, the GPR91 inhibitor is selected from the group consisting of:
2-(2-(4'-((4-methylpiperazin-1-yl)methyl)-[1,1'-biphenyl]-3-carboxamido)phenyl)acetic acid;
N-[{3-(3-trifluoromethyl-4-fluorophenyl)isoxazol-5-yl}methyl]-4-([1,8]naphthyridin-2-yl)butyramide;
2-(2-(4'-chloro-2,2'-difluoro-5'-(piperidin-3-yloxy)-[1,1'-biphenyl]-3-carboxamido)-5-fluorophenyl)acetic acid;
N-(1-(4'-fluoro-3'-(trifluoromethyl)-[1,1'-biphenyl]-4-yl)ethyl)-4-(1,8-naphthyridin-2-yl)benzamide;
2-[3-[4-(8-isoquinolyloxy)-2-(trifluoromethyl)phenoxy]phenyl]acetic acid;
2-[3-[4-phthalazin-5-yloxy-2-(trifluoromethyl)phenoxy]phenyl]acetic acid;
3-[3-[4-phthalazin-5-yloxy-2-(trifluoromethyl)phenoxy]phenyl]propanoic acid;
2-[3-[4-(8-chlorophthalazin-5-yl)oxy-2-(trifluoromethyl)phenoxy]phenyl]acetic acid;
2-[3-[4-(1-methylindazol-7-yl)oxy-2-(trifluoromethyl)phenoxy]phenyl]acetic acid;
2-[3-[4-(6-quinolyloxy)-2-(trifluoromethyl)phenoxy]phenyl]acetic acid;
3-[3-[4-imidazo[1,2-a]pyridin-8-yloxy-2-(trifluoromethyl)phenoxy]phenyl]propanoic acid;
2-[3-[4-[(5-fluoro-8-isoquinolyl)oxy]-2-(trifluoromethyl)phenoxy]phenyl]acetic acid;
2-[3-[4-quinazolin-5-yloxy-2-(trifluoromethyl)phenoxy]phenyl]acetic acid;
3-[3-[4-[(2-methyl-3,4-dihydro-1H-isoquinolin-8-yl)oxy]-2-(trifluoromethyl)phenoxy] phenyl]propanoic acid;
3-[3-[4-(2,3-dihydro-1,4-benzodioxin-5-yloxy)-2-(trifluoromethyl)phenoxy]phenyl]propanoic acid;
3-[3-[4-(8-quinolyloxy)-2-(trifluoromethyl)phenoxy]phenyl]propanoic acid;
3-[3-[4-(4-quinolyloxy)-2-(trifluoromethyl)phenoxy]phenyl]propanoic acid;
3-[3-[4-(5-isoquinolyloxy)-2-(trifluoromethyl)phenoxy]phenyl]propanoic acid;
2-[3-[4-(2-methylindazol-4-yl)oxy-2-(trifluoromethyl)phenoxy]phenyl]acetic acid;
2-[3-[4-(1-methylindazol-4-yl)oxy-2-(trifluoromethyl)phenoxy]phenyl]acetic acid;
2-[3-[4-([1,2,4]triazolo[4,3-a]pyridin-5-yloxy)-2-(trifluoromethyl)phenoxy]phenyl]acetic acid;
2-[3-[4-(3,4-dihydro-2H-1,4-benzoxazin-6-yloxy)-2-(trifluoromethyl)phenoxy]phenyl]acetic acid;
2-[3-[4-([1,2,4]triazolo [4,3-a]pyridin-7-yloxy)-2-(trifluoromethyl)phenoxy] phenyl] acetic acid;
2-[3-[4-[1-(oxetan-3-yl)indol-6-yl]oxy-2-(trifluoromethyl)phenoxy]phenyl]acetic acid;
2-[3-[4-[1-(oxetan-3-yl)indazol-6-yl]oxy-2-(trifluoromethyl)phenoxy]phenyl]acetic acid;
2-[3-[4-[2-(oxetan-3-yl)indazol-6-yl]oxy-2-(trifluoromethyl)phenoxy]phenyl]acetic acid;
2-[3-[4-[1-(oxetan-3-yl)indazol-5-yl]oxy-2-(trifluoromethyl)phenoxy]phenyl]acetic acid;
2-[3-[4-[2-(oxetan-3-yl)indazol-5-yl]oxy-2-(trifluoromethyl)phenoxy]phenyl]acetic acid;
2-[3-[4-[3-(oxetan-3-yl)benzotriazol-5-yl]oxy-2-(trifluoromethyl) phenoxy]phenyl]acetic acid;
2-[3-[4-[3-(oxetan-3-yl)benzimidazol-5-yl]oxy-2-(trifluoromethyl)phenoxy] phenyl]acetic acid;
2-[3-[4-[2-methyl-3-(oxetan-3-yl)benzimidazol-5-yl]oxy-2-(trifluoromethyl) phenoxy] phenyl]acetic acid;
2-[3-[2-(difluoromethyl)-4-[2-methyl-3-(oxetan-3-yl)benzimidazol-5-yl]oxy-phenoxy] phenyl]acetic acid;
2-[3-[4-[1-(oxetan-3-yl)imidazo[4,5-b]pyridin-6-yl]oxy-2-(trifluoromethyl) phenoxy] phenyl]acetic acid;
2-[3-[4-[1-(oxetan-3-yl)pyrrolo[2,3-b]pyridin-6-yl]oxy-2-(trifluoromethyl) phenoxy] phenyl]acetic acid;
6-[4-[3-(carboxymethyl)phenoxy]-3-(trifluoromethyl)phenoxy]imidazo[1,5-a]pyridine-3-carboxylic acid;
2-[3-[4-[1-(carboxymethyl)indol-5-yl]oxy-2-(trifluoromethyl)phenoxy]phenyl]acetic acid;
2-[3-[4-[1-(carboxymethyl)indol-6-yl]oxy-2-(trifluoromethyl)phenoxy]phenyl]acetic acid;
3-[4-[4-(8-isoquinolyloxy)-2-(trifluoromethyl)phenoxy]phenyl]propanoic acid;
3-[3-[4-(2-methylindazol-5-yl)oxy-2-(trifluoromethyl)phenoxy]phenyl]propanoic acid;
2-[3-[[5-(8-isoquinolyloxy)-3-(trifluoromethyl)-2-pyridyl]oxy]phenyl]acetic acid;
3-[3-[4-imidazo[1,2-a]pyridin-5-yloxy-2-(trifluoromethyl)phenoxy]phenyl]propanoic acid;
2-[3-[2-cyclopropyl-4-(8-isoquinolyloxy)phenoxy]phenyl]acetic acid;
2-[3-[2-cyclopropyl-4-[2-methyl-3-(oxetan-3-yl)benzimidazol-5-yl]oxy-phenoxy] phenyl]acetic acid;
2-[3-[4-[(1-chloro-8-isoquinolyl)oxy]-2-(trifluoromethyl)phenoxy]phenyl]acetic acid;
2-[3-[4-[(1-methyl-8-isoquinolyl)oxy]-2-(trifluoromethyl)phenoxy]phenyl]acetic acid;
3-[3-[4-(8-isoquinolyloxy)-2-(trifluoromethyl)phenoxy]phenyl]propanoic acid;
3-[3-[4-(1,2,3,4-tetrahydroisoquinolin-8-yloxy)-2-(trifluoromethyl) phenoxy] phenyl] propanoic acid;
3-[3-[4-indan-1-yloxy-2-(trifluoromethyl)phenoxy]phenyl]propanoic acid;
3-[3-[4-(1H-indazol-5-yloxy)-2-(trifluoromethyl)phenoxy]phenyl]propanoic acid;
2-[3-[4-[(3-cyano-1H-indazol-5-yl)oxy]-2-(trifluoromethyl) phenoxy]phenyl]acetic acid;
5-[4-[3-(carboxymethyl)phenoxy]-3-(trifluoromethyl)phenoxy]-1H-indazole-3-carboxylic acid;
2-[3-[4-(1H-indazol-4-yloxy)-2-(trifluoromethyl)phenoxy]phenyl]acetic acid;
2-[3-[4-imidazo[1,5-a]pyridin-5-yloxy-2-(trifluoromethyl)phenoxy]phenyl]acetic acid;
2-[3-[4-imidazo[1,5-a]pyridin-8-yloxy-2-(trifluoromethyl) phenoxy]phenyl]acetic acid;
2-[3-[4-(3-methylimidazo[1,5-a]pyridin-5-yl)oxy-2-(trifluoromethyl)phenoxy]phenyl]acetic acid;
2-[3-[4-(3-methylimidazo[1,5-a]pyridin-8-yl)oxy-2-(trifluoromethyl)phenoxy]phenyl]acetic acid;
2-[3-[4-[3-(oxetan-3-yl)imidazo[1,5-a]pyridin-6-yl]oxy-2-(trifluoromethyl) phenoxy] phenyl] acetic acid;
2-[3-[4-(8-isoquinolyloxy)-2-(trifluoromethyl)phenoxy]phenyl]-N-methylsulfonyl-acetamide;
3-[3-[4-(8-isoquinolyloxy)-2-(trifluoromethyl)phenoxy]phenyl]propanamide;
2-[3-[4-(8-isoquinolyloxy)-2-(trifluoromethyl)phenoxy]phenyl]acetamide;
8-[4-[3-[2-(4H-1,2,4-triazol-3-yl)ethyl]phenoxy]-3-(trifluoromethyl)phenoxy]isoquinoline;
8-[4-[3-[2-(1H-tetrazol-5-yl)ethyl]phenoxy]-3-(trifluoromethyl)phenoxy]isoquinoline;
8-[4-[3-(1H-tetrazol-5-ylmethyl)phenoxy]-3-(trifluoromethyl) phenoxy]isoquinoline; and
2-[3-[4-(8-isoquinolyloxy)-3-(trifluoromethyl)phenoxy]phenyl]acetic acid;
or a pharmaceutically acceptable salt thereof.

In one embodiment, the inhibitor of GPR91 is selected from the group consisting of:
2-(2-(4'-((4-methylpiperazin-1-yl)methyl)-[1,1'-biphenyl]-3 - carboxamido)phenyl)acetic acid having a structure represented by 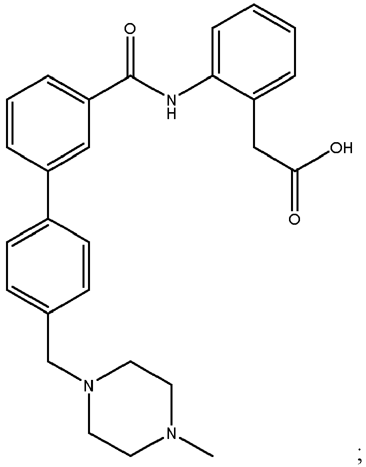
N-[{3-(3-trifluoromethyl-4-fluorophenyl)isoxazol-5-yl}methyl]-4-([1,8]naphthyridin-2-yl)butyramide) having a structure represented by 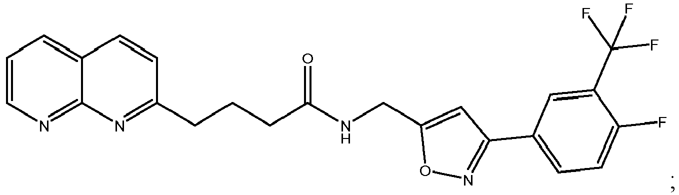 or
a pharmaceutically acceptable salt thereof.

These compounds may be synthesised according to the methods set out in Bhuniya D. et al., Discovery of a potent and selective small molecule hGPR91 antagonist, Bioorganic & Medicinal Chemistry Letters, Volume 21, Issue 12, 15 June 2011, Pages 3596-3602, doi.org/10.1016/j.bmcl.2011.04.091, or the methods set out in Haffke M. et al., Structural basis of species-selective antagonist binding to the succinate receptor. Nature 574, 581-585 (2019). https://doi.org/10.1038/s41586-019-1663-8, with suitable modification by a person skilled in the art.

GPR91 inhibition can be measured according to the art. For example, GPR91 inhibition may be measured by testing a compound in a G-protein functional assay such as Calcium flux, IP1 accumulation and GTPyS. GPR91 inhibition may be measured by testing a compound in phenotypic assays which measures a cytokine response that is relevant to the indication.

In one embodiment, the GPR91 inhibitor comprises a nucleic acid molecule. In one embodiment, the nucleic acid molecule is a ribonucleic acid (RNA) or deoxyribonucleic acid (DNA) molecule that inhibits GPR91. The person skilled in the art would be readily capable of identifying nucleic acid molecules that inhibit GPR91. In one embodiment, the GPR91 inhibitor is an antibody or fragment thereof that is capable of inhibiting GPR91. Antibodies that inhibit GPR91 are known to the skilled person. For example, the antibody may be one that immunospecifically binds a polypeptide, polypeptide fragment, or variant of SEQ ID NO. 3, and/or an epitope of GPR91, or an extracellular domain fragment of GPR91.

The term "antibody" as used herein refers to immunoglobulin molecules and immunologically active portions of immunoglobulin molecules that contain an antigen binding site that immunospecifically binds an antigen. The immunoglobulin molecules of the invention can be of any type such as IgG, IgE, IgM, IgD, IgA and IgY, any class such as IgG1, IgG2, IgG3, IgG4, IgAl and IgA2, and any subclass of immunoglobulin molecule. Moreover, the term "antibody" (Ab) or "monoclonal antibody" (Mab) is meant to include intact molecules and antibody fragments (such as, for example, Fab and F(ab')2 fragments) which are capable of specifically binding to protein. Fab and F(ab')2 fragments lack the Fc fragment of intact antibody, clear more rapidly from the circulation of an animal, and may have less non-specific tissue binding than an intact antibody. Moreover, antibodies used in the present disclosure include chimeric, single chain, and humanized antibodies.

In one embodiment, the GPR91 inhibitor is a binding protein.

In one embodiment, the GPR91 inhibitor is an aptamer.

Methods of screening for compounds that modulate GPR91 activity are known in the art. For example, the method of screening for compounds that modulate GPR91 activity comprises:
1) Preparing a transfected cell (for example, a mast cell) comprising a nucleic acid sequence that encodes the amino acid sequence of SEQ ID NO3;
2) Contacting the transfected cell or cells with at least one test compound;
3) monitoring said cell for a compound that modulates GPR91's activity.

### HAIR-LOSS RELATED DISORDERS

The present disclosure provides for the treatment of hair-loss related disorders such as alopecia areata, alopecia universalis, alopecia totalis, androgenetic alopecia, and telogen effluvium.

Alopecia Areata (AA) is a highly prevalent autoimmune disease that leads to hair loss due to the collapse of immune privilege of the hair follicle. AA affects hair follicles in the anagen (growth) phase of the hair cycle. Patients affected with AA typically regrow hair in patches with a loss of color or white in color. AA is characterized with quick onset, and typically occurs in times of stress. The whitening of hair follicles is believed to result from an acute attack upon the pigmented hair follicles.

Alopecia universalis (AU) is the most severe form of AA and involves the loss of all body hair including eyebrows, eyelashes, chest hair, armpit hair, and pubic hair. Similarly, Alopecia totalis is the loss of all hair on the head and face.

Androgenetic alopecia is a common type of hair loss in both men and women. This is generally known as male pattern baldness (MPB) in men and female pattern hairloss (FPHL) in women, and the terms Androgenetic alopecia and MPB/FPHL are used interchangeably. In male subjects suffering from AGA, hair is lost in a well-defined pattern beginning above both temples. Over time, the hairline recedes to form a characteristic "M" shape. Hair also thins at the crown of the head and generally progresses to partial or complete baldness. In females, the frontal hairline is typically spared and FPHL instead presents clinically as diffuse hair thinning across the scalp. Various classification systems have been developed to codify the the severity of hair loss. One example is the Hamilton-Norwood scale. This ranges from stages I (least severe) to VII (most severe). The Hamilton-Norwood scale is described in D. Norwood, Male Pattern Baldness: Classification and Incidence, South. Med. J. 68(11):1359-65 (1975). Another example is the Ludwig classification used to assess the progression of androgen dependent female pattern hairloss as described in Ludwig E. Classification of the types of androgenetic alopecia (common baldness) occurring in the female sex. Br J Dermatol. 1977;97:247-54.

Telogen effluvium is a scalp disorder characterized by the thinning or shedding of hair resulting from the early entry of hair in the telogen phase (the resting phase of the hair follicle).

In one embodiment, the subject in need thereof is male and has stage I male pattern baldness. In one embodiment, the subject in need thereof is male and has stage II male pattern baldness. In one embodiment, the subject in need thereof is male and has stage III male pattern baldness. In one embodiment, the subject in need thereof is male and has stage III vertex male pattern baldness. In one embodiment, the subject in need thereof is male and has stage IV male pattern baldness. In one embodiment, the subject in need thereof is male and has stage V male pattern baldness. In one embodiment, the subject in need thereof is male and has stage VI male pattern baldness. In one embodiment, the subject in need thereof is male and has stage VII male pattern baldness. In one embodiment, the subject in need thereof is male and has stage IIA male pattern baldness. In one embodiment, the subject in need thereof is male and has stage IIIA male pattern baldness. In one embodiment, the subject in need thereof is male and has stage IVA male pattern baldness. In one embodiment, the subject in need thereof is male and has stage VA male pattern baldness.

In one embodiment, the subject in need thereof is female and has stage I female pattern baldness. In one embodiment, the subject in need thereof is female and has stage II female pattern baldness. In one embodiment, the subject in need thereof is female and has stage III female pattern baldness.

### PHARMACEUTICAL COMPOSITIONS/ROUTES OF ADMINISTRATION/DOSAGES

An inhibitor of GPR91 may be administered by any convenient route. In particular embodiments, the inhibitor of GPR91 may be administered by topically, orally, parenterally, intranasally or by inhalation. In one embodiment, the inhibitor of GPR91 is administered in a pharmaceutical composition. In one embodiment, the inhibitor of GPR91 is formulated in a pharmaceutical composition adapted for oral or parenteral administration, or for administration intranasally or by inhalation. In one embodiment, appropriate doses will readily be appreciated by those skilled in the art.

According to one aspect, the disclosure provides a pharmaceutical composition comprising an inhibitor of GPR91 and a pharmaceutically acceptable excipient. According to another aspect, the disclosure provides a process for the preparation of a pharmaceutical composition comprising admixing an inhibitor of GPR91 with a pharmaceutically acceptable excipient.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for nasal administration can comprise a coarse powder having a particle size for example in the range 20 to 500 microns which is administered in the manner in which snuff is taken, *i.e.,* by rapid inhalation through the nasal passage from a container of the powder held close up to the nose. Suitable formulations wherein the carrier is a liquid, for administration as a nasal spray or as nasal drops, include aqueous or oil solutions of the inhibitor of GPR91.

Pharmaceutical formulations adapted for administration by inhalation include fine particle dusts or mists, which may be generated by means of various types of metered, dose pressurized aerosols, nebulizers or insufflators.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations described herein may include other agents conventional in the art having regard to the type of formulation in question, for example those suitable for oral administration may include flavouring agents.

The present disclosure also provides unitary pharmaceutical compositions in which the inhibitor of GPR91 and one or more other therapeutic agent(s) may be administered together. When an inhibitor of GPR91 is used in combination with a second therapeutic agent, the dose of each therapeutic agent may differ from the dose of that therapeutic agent when used alone.

In one embodiment, the disclosure provides a pharmaceutical composition comprising an inhibitor of GPR91 and at least one other therapeutic agent selected from: androgen receptor antagonist, JAK inhibitor, minoxidil, finasteride, dutasteride, or anti androgens.

In one embodiment, the JAK inhibitor is selected from tofacitinib, ruxolitinib, baricitinib, and momelotinib. In one embodiment, the JAK inhibitor is ruxolitinib.

In one embodiment, the androgen receptor antagonist is selected from flutamide, bicalutamide, spironolactone, cyproterone acetate, marijuana and nilutamide. In one embodiment, the androgen receptor antagonist is selected from flutamide, bicalutamide, and nilutamide.

In one embodiment, the disclosure provides a pharmaceutical composition comprising an inhibitor of GPR91 and at least one other therapeutic agent selected from: minoxidil, finasteride, dutasteride, or antiandrogens.

In one embodiment, the inhibitor of GPR91 is formulated for topical administration.

### THERAPEUTIC USE

The present disclosure provides the use of an GPR91 inhibitor in the treatment or prevention of hair-loss related disorder or a symptom thereof in a subject in need thereof. In one embodiment, the hair-loss related disorder is selected from alopecia areata, alopecia universalis, alopecia totalis, androgenetic alopecia, and telogen effluvium. In one embodiment, the hair-loss related disorder is androgenetic alopecia.

The present disclosure provides the use of an GPR91 inhibitor in the treatment or prevention of androgenetic alopecia or a symptom thereof in a subject in need thereof.

The present disclosure provides a method for treating or preventing androgenetic alopecia in a subject in need thereof, comprising administering a therapeutically effective amount of a GPR91 inhibitor.

The term "prevent" or "preventing" as used herein includes preventing the severity or frequency of a symptom of a hair-loss related disorders or a symptom thereof. The term "treatment" or "treating" refers to ameliorating or stabilizing the specified condition, reducing or eliminating the symptoms of the condition, slowing or eliminating the progression of the condition, and preventing or delaying reoccurrence of the condition in a previously afflicted patient or subject. The term "subject" refers to a human unless otherwise stated.

In one embodiment, the present disclosure provides a method for treating or preventing a hair-loss related disorder (such as alopecia areata, alopecia universalis, alopecia totalis, androgenetic alopecia, and telogen effluvium) or a symptom thereof in a subject in need thereof comprising the steps of:
a) identifying a subject with or at risk of developing a hair-loss related disorders or a symptom thereof; and
b) administering a therapeutically effective amount of an inhibitor of GPR91 to the subject;
whereby the hair-loss related disorders or a symptom thereof in the subject is treated.

In one embodiment, the present disclosure provides a method for treating or preventing androgenetic alopecia in a subject in need thereof comprising the steps of:
a) identifying a subject with or at risk of developing androgenetic alopecia; and
b) administering a therapeutically effective amount of an inhibitor of GPR91 to the subject;
whereby the androgenetic alopecia in the subject is treated.

In some embodiments, the subject is afflicted with or is at risk of developing a hair-loss related disorders or a symptom from an underlying health disorder or genetic disposition, such as cardiovascular disease, insulin resistance and obesity.

In some embodiments, the subject is afflicted with or is at risk of developing alopecia from an underlying health disorder or genetic disposition, such as cardiovascular disease, insulin resistance and obesity.

In one embodiment, the present disclosure provides an inhibitor of GPR91 for use in inducing or promoting hair growth in a human. In another embodiment, the disclosure provides a method of inducing or promoting hair growth in a human, the method comprising administering an effective amount of an inhibitor of GPR91. In one embodiment, inducing or promoting hair growth in a human means inducing a transition from a telogen phase to an anagen phase of a hair growth cycle. In one embodiment, the inhibitor of GPR91 increases a number of hair follicles present in a subject in the anagen phase.

In one embodiment, the inhibitor of GPR91 increases the size of the dermal papilla in a patient.

In one embodiment, the method of the present disclosure further comprises administration of an effective amount of minoxidil, finasteride, dutasteride, antiandrogens, hair transplantation procedures, prostaglandin analogues, light therapy, platelet-rich plasma, or micro needling.

In one embodiment, the use of the present disclosure further comprises administration of an effective amount of minoxidil, finasteride, dutasteride, antiandrogens, hair transplantation procedures, prostaglandin analogues, light therapy, platelet-rich plasma, or micro needling.

In one embodiment, the present disclosure provides the use of an inhibitor of GPR91 in the manufacture of a medicament for use in the treatment or prevention of a hair-loss related disorder or a symptom thereof. In one aspect of this embodiment, the hair-loss related disorder is selected from alopecia areata, alopecia universalis, alopecia totalis, androgenetic alopecia, and telogen effluvium. In one aspect of this embodiment, the hair-loss related disorder is of androgenetic alopecia.

### EXAMPLES

### Example 1: GPR91/SUCNR1 expression in hair follicle relevant cell types (mast cells, macrophages, hair follicle keratinocytes and dermal papilla)

The expression of GPR91 in immune cells known to reside in the perifollicular space (mast cells and macrophages) and hair follicle relevant cells (dermal papilla and hair follicle keratinocytes) was assessed.

### Cell culture:

IPSC precursor cells were differentiated into macrophages using differentiation media known in the art followed by polarisation into M1 and M2 macrophage phenotypes using LPS + IFN-γ and IL4 + IL13 stimulation, respectively.

U937 and HEK293 cells were obtained from ATCC, LUVA cells were obtained from Kerafast Inc. LAD2 cells were obtained from the NIH. Dermal papilla cells were obtained from Cell Applications Inc. Human hair follicle keratinocyte mRNA was purchased from Sciencell.

### mRNA expression

mRNA was extracted from the relevant cell line using the MAGMAX^{®} 96 Total RNA isolation kit (Applied Biosystems) and Kingfisher equipment in accordance with the manufacturer's protocol. mRNA was quantified using a multi spectrophotometer. RT-PCR was performed using a High Capacity reverse transcription kit (Applied Biosystems). qPCR was performed using a ABI QUANTSTUDIO^{®} 7 Flex (Applied Biosystems). A GPR91 primer probe set used to determine expression levels was purchased from IDT (Hs.PT.58.28046518) Ref Seq number: NM_033050(1). Relative expression (AU: Arbritrary units) was calculated using 2^{-dCT}, where dCT is calculated as CT (GPR91) - CT(Housekeeper Gene). In these experiments, the housekeeper gene used was B2M (β-2 microglobulin) (Matsuzaki Y et al., Regenerative Therapy, Volume 1, June 2015, Pages 91-97).

### Protein expression

Cell surface expression of GPR91 was performed by flow cytometry using a Flow cytometer. Flow cytometry is performed in a 96-well plate format with 1 million cells per well for each condition. Cells are blocked with human Fc blocking receptor (2uL) for 10 minutes at room temperature. After blocking, cells are incubated with antibodies for 30 minutes at 4 degrees Celsius. Cells are then washed and fixed using a fixation solution (Biolegend) for 15 minutes at room temperature. The antibody used to detect GPR91 was purchased from Alomone labs (ASR-090-F, Anti-GPR91 (SUCNR1) Extracellular-FITC, 1 mg/ml). For each condition, 2.5 µL of antibody is used at a concentration of 2.5 µg. The isotype control antibody used in this experiment is Rabbit IgG (FITC) from eBioscience.

### Results

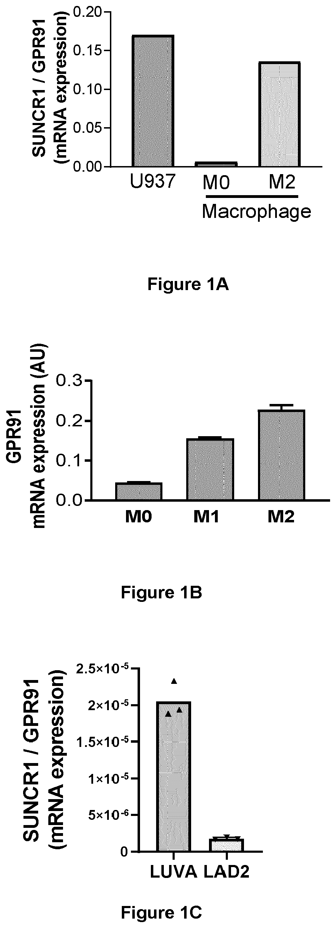

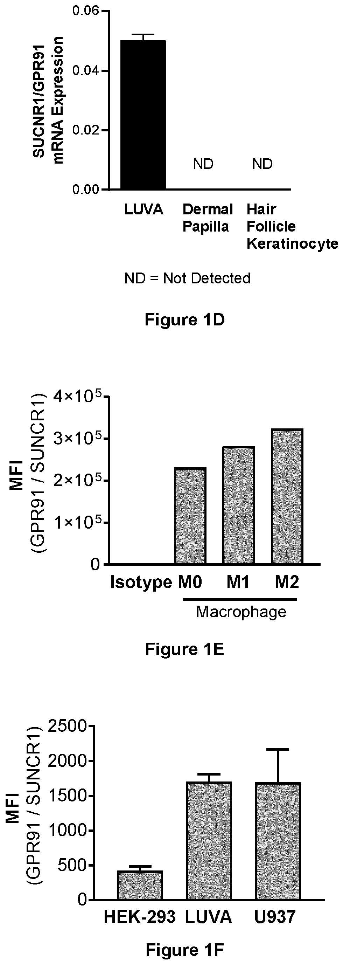

The results are shown in Figures 1A, 1B, 1C (mRNA expression), and 1D and 1E (protein expression). These results demonstrate that GPR91 is highly expressed in immune cell types that are relevant to the hair follicle perifollicular region and are demonstrated to play a role in alopecia. These results also demonstrate the GPR91 is not expressed in hair follicle cells.

### Example 2: Effect of succinate stimulation in the secretion of proinflammatory mediators

The effect of succinate stimulation on LUVA cells was assessed to identify the role of GPR91.

### Succinate pEC50:

Sodium succinate was diluted directly into cell culture media (StemPro-34 SFM + nutrition supplement + glutamine + Pen strep). Succinate, in serial dilutions, was incubated with the LUVA cells for 3 hours. After the incubation, the cell suspension was centrifuged, and the supernatant was collected for downstream readouts of secreted factors including Prostaglandin D2 (PGD2) by ELISA (Cayman Chemical PGD2 EIA kit), and cytokines/chemokines TNFa, MIP1a and Eotaxin-3 by MSD (Mesoscale V-Plex Plus proinflammatory panel 1, Plus Chemokine Panel 1).

### GPR91 Knockout cell line development:

A GPR91 knockout in LUVA cells (received from Kerafast Inc) was generated using CRISPR technology with a single guide following protocols known in the art. The guide RNA sequence used was GUAGUAGCCGUAAACAACAA. The guide RNA cut location was chromosome 3: 151,880,674.

### Compound pIC50:

Cells were pre-treated (at a density of 1e6) for PGD2 and with compound 1 (2-(2-(4'-((4-methylpiperazin-1-yl)methyl)-[1,1'-biphenyl]-3-carboxamido)phenyl)acetic acid), compound 2 (N-[{3-(3-trifluoromethyl-4-fluorophenyl)isoxazol-5-yl}methyl]-4-([1,8]naphthyridin-2-yl)butyramide),or vehicle (DMSO at 0.1%) for 1hr prior to succinate stimulation.

Compound 1 can be synthesised according to methods known in the art, and has the below structure:

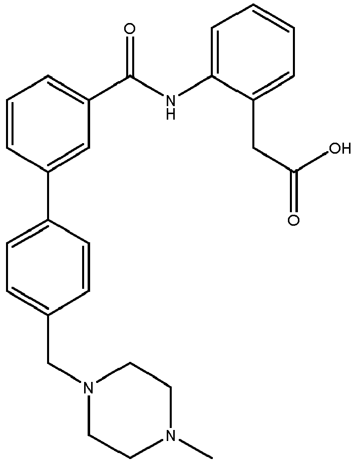

Compound 2 can be synthesised according to methods known in the art, and has the below structure:

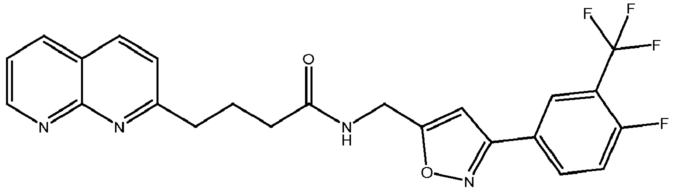

### Results

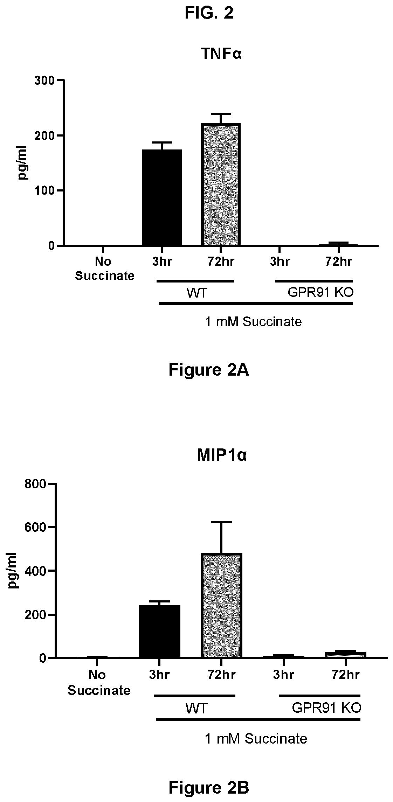

The results in Table 2 show that succinate stimulation of LUVA cells drives secretion of prostaglandin D2 (PGD2) and proinflammatory cytokines/chemokines associated with AGA, and that GPR91 inhibitors are able to inhibit prostaglandin D2 (PGD2) and proinflammatory cytokine/chemokine secretion. The specificity of the succinate response for GPR91 is shown in Figure 2A and 2B. In particular, at both 3hr and 72 hour post succinate addition, a robust TNFa and MIP-1a response is produced. At the same time points in a GPR91 knockout LUVA cell line, there is a lack of response to succinate indicating that the cytokine response is specific to GPR91.

**Table 2**

| **Analyte** | **Succinate control pEC₅₀** | **Test compound pIC₅₀** | |
|---|---|---|---|
| | | **Compound 1** | **Compound 2** |
| Calcium flux in HEK293 | | 7.4 | 7.6 |
| GTPyS | | 7.5 | 5.9 |
| IP1 | | 7.0 | 5.1 |
| PGD2 | 3.5 | 7.6 | 7.2 |
| TNF-α | 4.2 | | |
| MIP-1a | 3.4 | 7.1 | 6.0 |

### Example 4: GPR91/SUCNR1 expression in physiologically relevant tissue from mouse and human samples.

The expression of GPR91 in mouse skin and human hair follicles were assessed using the following method.

### Mouse model with humanized immune system:

A humanized mouse model was aquired from Taconic Biosciences. In brief, a humanized immune system was generated by engrafting human CD34+ hematopoetic stem cells (HSC) into an immunodeficient NOG mouse (NOG-EXI, strain) expressing human GM-CSF and human IL-3 cytokines to support myeloid lineage engraftment. Mice with >25% human CD45+ present in blood and greater than 10 weeks post engraftment were used for further analysis.

### Flow cytometry

To profile expression of human GPR91 in mouse skin immune cells, skin was collected and pooled from 3 mice for each HSC donor and placed on ice in PBS. Subcutaneous fat was removed by scraping followed by trypsinization (0.25% for 20 minutes at 37 degrees Celsius) to remove the epidermis. Hair and epidermis were then removed by scraping. The tissue was then digested into a single cell suspension using 3mg/ml liberase (Roche) solution at 37 degrees Celsius. Flow cytometry was performed to detect cell surface expression of GPR91 as described in example 1. To detect GPR91 protein expression we conjugated compound 1 (as described previously) to a fluorescent probe (Alexa-488 or Alexa-647). For the cell gating and cellular phenotyping the following antibodies from BioLegend were used: anti-CD45 (mouse clone 30-F11 and human clone **2D1**), anti-CD14 (clone HCD14) , anti-HLA-DR (clone I243), anti-CD117 (clone QA18A19), anti-FceRIa (clone AER-37).

### Immunofluorescence on human hair follicles.

Hair follicle samples from elective surgeries have been obtained after informed, written patient consent under ML biobank approval (2019-297-f-S) and processed for further analysis under the ethics committee study approval (2020-945-f-S) or ethical approval by University of Muenster (2015-602-f-S) and the Comite' de Bioetica de la Universidad Fernando Pessoa Canarias (03(2020-06-22)). All studies on human hair follicles were performed at Monasterium Laboratories Skin and Hair Research Solutions GMBH.

Hair follicle organ cultures *ex vivo:* Full length hair follicles were microdissected from scalp skin or Follicular unit extraction (FUE) as described in Edelkamp et al., Molecular Dermatology 2020, Langan et al., Exp Dermatol 2015. GPR91 expression in human hair follicles was performed using the fluorescent tool described previously or the anti-GPR91 antibody described in example 1.

For detection of human GPR91 with the Alexa conjugated tool, two methods were used. Scalp skin biopsies/microdissected hair follicles were co-cultured with the tool or sections of hair follicle from scalp skin biopsies/microdissection were incubated with the tool. To detect GPR91 using the anti-GPR91 antibody described previously, sections were fixed with PFA for 20 mins, followed by blocking with 10% BSA for 20 mins. The primary antibody (anti-GPR91) was applied overnight followed by a secondary antibody (Donkey anti-Rabbit Alexafluor488) for 45 mins. Sections were stained with DAPI to visualize the nucleus and images were taken using a high resolution fluorescent microscope (Keyence Biozero) and fluorescence was detected using the appropriate filters.

### Results

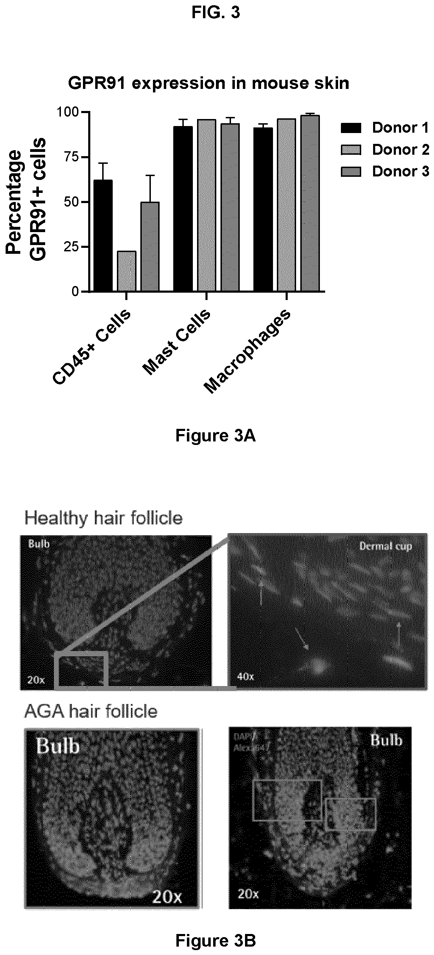

The results are shown in Figures 3A (human immune cells isolated from mouse skin) and 3B (human hair follicles). These results demonstrate that in the physiologically relevant context (skin/scalp) GPR91 is highly expressed in immune cell types that are relevant to the hair follicle and are demonstrated to play a role in alopecia.

### Example 4: Succinate increases TREM2 expression in iPSC-derived macrophages

The effect of succinate on TREM2 expression in macrophages was assessed using the following method.

IPSC precursor cells were differentiated into macrophages using differentiation media known in the art followed by polarisation into M1 and M2 macrophage phenotypes using LPS + IFNg and IL4 + IL13 stimulation, respectively. Succinate treatment was added during the polarisation process concurrent with the cytokines listed previously.

mRNA was extracted using the method described in Example 1. The TREM2 primer probe set used to determine expression levels was purchased from ThermoFisher (Hs01003898_m1). Relative expression (AU: Arbitrary units) was calculated using 2^{-dCT}, where dCT is calculated as CT (TREM2) - CT(Housekeeper Gene). In these experiments, the housekeeper gene used was RPLP0 (ribosomal phosphoprotein PO) (Primer Probe purchased from ThermoFisher Assay ID: He00420895_gH).

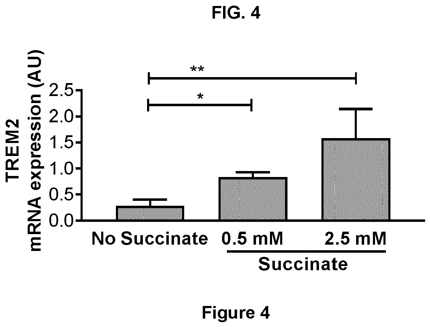

The results are shown in Figure 4. These results demonstrate that with the presence of succinate during macrophage polarization, particularly when skewing towards an M2 phenotype, hair follicle niche specific reprogramming generates a unique subset of macrophages that are marked by an upregulation of TREM2.

### Example 5: Succinate-treated TREM2 positive iPSC-derived macrophages secrete higher levels of Oncostatin M

The effect of succinate on Oncostatin M levels was assessed using the following method.

iPSC derived macrophages were polarised into an M2 phenotype by stimulating the cells with IL4 and IL13, as described previously, in the presence or absence of the indicated concentrations of succinate. Cell culture supernatants were collected at 5 and 48 hours post addition of polarising cytokines +/- succinate to determine the levels of Oncostatin M secreted by the cells. Oncostatin M levels were determined by ELISA (Abcam ab215543) according to the manufacturer's instructions.

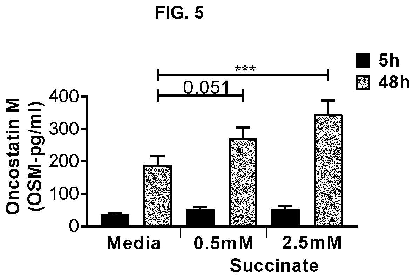

The results are shown in Figure 5. Figure 5 demonstrates that disease relevant TREM2 positive iPSC derived M2 macrophages secrete higher levels of Oncostatin M (a cytokine that has shown to induce hair follicle quiescence in mice), when treated with succinate.

### Example 6: TREM2 positive iPSC derived M2 macrophages secrete Oncostatin M in a GPR91 dependent manner

The effect of GPR91 inhibitors on Oncostatin M secretion by TREM2 positive iPSC macrophages was assessed using the following method.

Two different donors of iPSC derived macrophages were polarized into an M2 phenotype by treating the cells with IL4 and IL-13 for 48-96 hours (as indicated), as described previously, in the presence or absence of the indicated concentrations of succinate. Cell culture supernatants were collected post addition of polarizing cytokines +/succinate to determine the levels of Oncostatin M secreted by the cells. Oncostatin M levels were determined by ELISA according to the manufacturer's instructions. To determine the effect of GPR91 inhibition, 10uM of Compound 1 (at 0.1% DMSO) was added to the cell culture 2.5 hours prior to succinate addition. Supernatants were collected and Oncostatin M levels were measured.

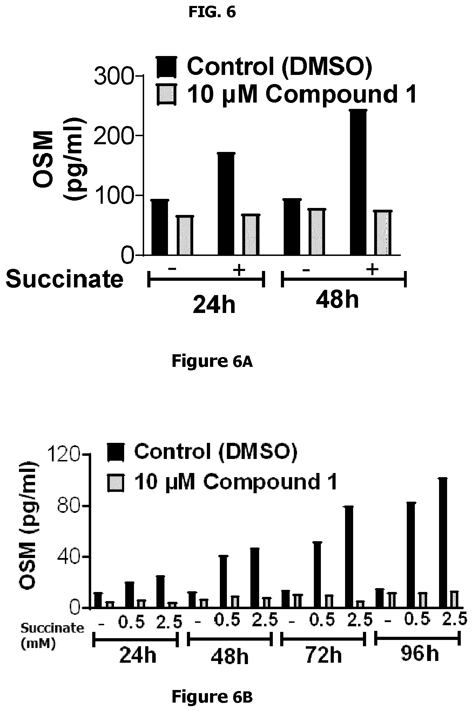

The results are shown in Figure 6. The results demonstrate that inhibiting GPR91 significantly reduce the levels of Oncostatin M secreted by succinate treated TREM2 positive iPSC derived macrophages.

### Example 7: iPSC derived M2 macrophages treated with succinate induce secreted factors that inhibit dermal papilla proliferation in a GPR91/SUCNR1 dependent manner

The effect of GPR91 inhibition on iPSC derived M2 macrophages treated with succinate and how this could impair dermal papilla cell proliferation was assessed using the following method.

iPSC were differentiated and polarized to an M2 like macrophage phenotype as previously described. In this experiment, during the polarization step, 3 mM succinate was applied in the presence or absence of 10 µM GPR91 inhibitor (Compound 1) added 2.5 hours prior to succinate addition. Media was collected from the treated iPSC after 48 hours of incubation, which is referred to as conditioned media. The dermal papilla from both male and female donors were plated at 5K cells/well onto a collagen coated E-Plate and analysed using the XCELLIGENCE^{®} platform according to the manufacturer's instructions. The cells are analysed in the machine for 24 hours to determine adherence to the plate and establishment of baseline measurements. After 24 hours, the media is aspirated, and macrophage conditioned media is added to the cells for up to 117 hours and the effects of the GPR91 dependent iPSC secretome on dermal papilla proliferation are analyzed.

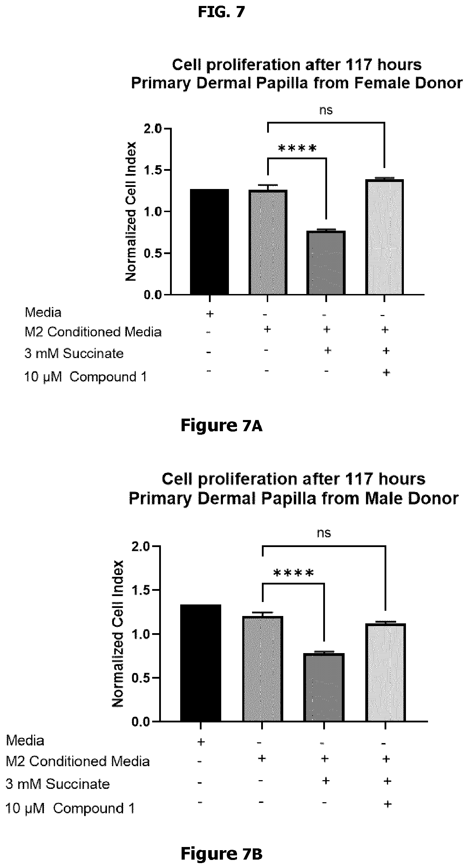

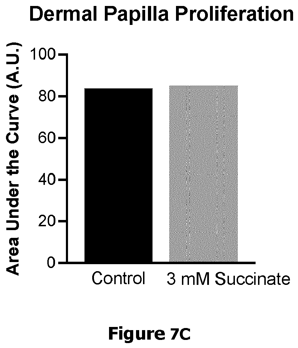

The results are shown in Figures 7A and 7B which demonstrate that M2 disease associated macrophages when treated with succinate induce secreted factors that inhibit dermal papilla proliferation (from both male and female donors), and that these deleterious secreted factors can be reverted by the administration of a GPR91 inhibitor.

### Example 8: Validation in human dermal papilla cells that Oncostatin M activates the JAK-STAT pathway and induces senescence.

The downstream effects of recombinant oncostatin M was assessed by evaluating signalling pathways (such as the JAK-STAT pathway previously associated with oncostatin M induced senescence in mouse [Wang 2019, PMID 30930146]) and markers associated with senescence to link specific mediators in the macrophage secretome (like oncostatin M) to disease relevant mechanisms. Dermal papilla cells in culture were treated with recombinant oncostatin M protein from BioLegend. Tofacitinib was received from MedChemExpress as a ready-made 10 mM solution and used at the indicated concentration.

Protein quantification: protein was extracted after lysis with RIPA buffer and quantified by BCA using methods known in the art. Protein was loaded onto a gel and run with NuPage MES SDS running buffer and transferred onto nitrocellulose membrane using the iBlot2 according to manufacturer's instructions and procedures known in the art. The following primary antibodies were used to assess expression of the relative proteins described: Human anti-Total STATS antibody, Isotype (Rabbit polyclonal IgG) from Abcam, Human anti-Phospho STATS antibody, Isotype (Rabbit polyclonal IgG) from Abcam, Anti-Human total STAT3 (D3Z2G) from Cell Signaling Technologies, Anti-Human pSTAT3 (Y705) (D3A7) from Cell Signaling Technologies and the control antibody, β-Actin (8H10D10) Mouse IgG2b mAb from Cell Signaling Technologies. The following secondary antibodies were used: Donkey Anti-Mouse Antibody (for Control Antibody) and Goat Anti-Rabbit Antibody (for Target Antibody) from Cell Signaling Technologies.

Gene expression: mRNA was extracted as previously described in example 1. RT-qPCR was performed according to methods known in the art to detect genes that are up or downregulated after oncostatin M treatment (at 10 ng/ml and 100 ng/ml) that are known to play a deleterious role in hair follicles. The following Taqman probes were used to identify target specific mRNA transcripts: FGF18 (Assay ID: Hs00826077_m1), and BMP6 (Assay ID: Hs01099594_m1).

Beta Galactosidase Activity: A B-Galactosidase Activity Assay Kit (Fluorescence, Plate-Based) from Cell Signalling Technology #23833 was used to assess cellular senescence. The instructions were followed according to the manufacturer's instructions.

### Results

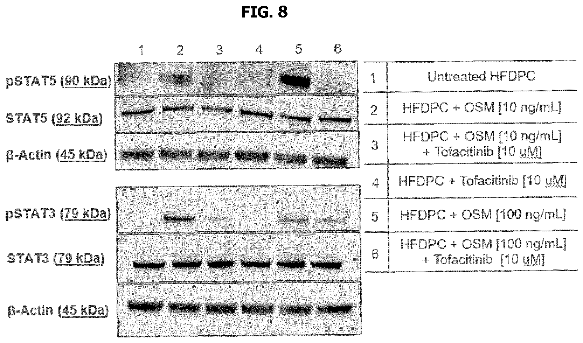

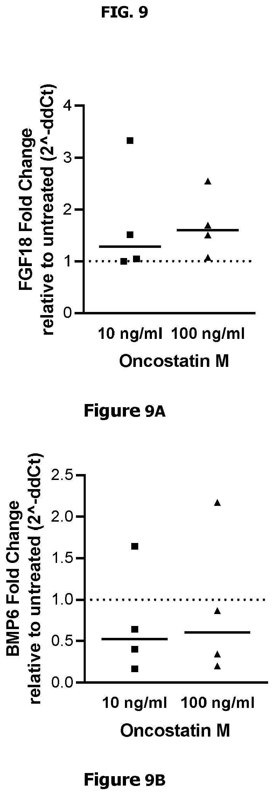

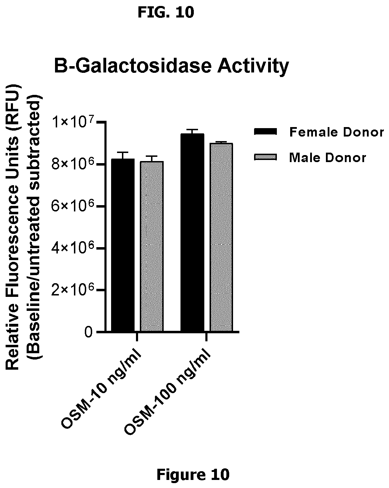

The results are shown in Figure 8, Figures 9A and 9B, and Figure 10 and demonstrate that oncostatin M, which is known to be secreted from succinate stimulated macrophages, activates the JAK-STAT signalling pathway in dermal papilla cells. This leads to the induction of senescence and the upregulation of genes that are known to maintain hair follicle quiescence and restrict the inductivity of the hair follicle.

### Example 9: Effect of GPR91/SUCNR1 modulation on healthy human hair follicle cycling

Hair follicle samples from elective surgeries have been obtained and processed for further analysis using the following method. Studies were conducted according to the Declaration of Helsinki Principles.

Hair follicle organ cultures *ex vivo:* Full length hair follicles were microdissected from scalp skin or Follicular unit extraction (FUE) as described in Edelkamp et al., Molecular Dermatology 2020, Langan et al., Exp Dermatol 2015.

Microscopic hair cycle staging: Hair cycle staging is performed at the end of the culture and can be determined according to several established parameters as describes in Langan et al., Exp Dermatol 2015. Hair follicles are microscopically evaluated for the hair cycle staging using Masson-Fontana histochemistry and Ki-67/TUNEL immunostaining as previously described in Kloepper et at., Exp Dermatol 2010; Langan et al., Exp Dermatol 2015. Compounds, at the concentrations indicated, are present throughout the duration of the hair follicle culture at 0.1% DMSO.

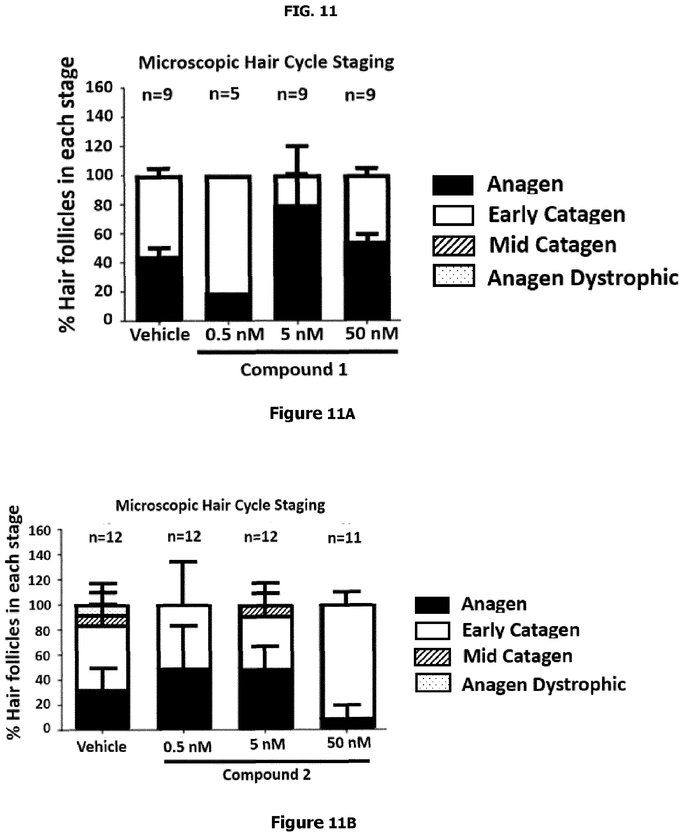

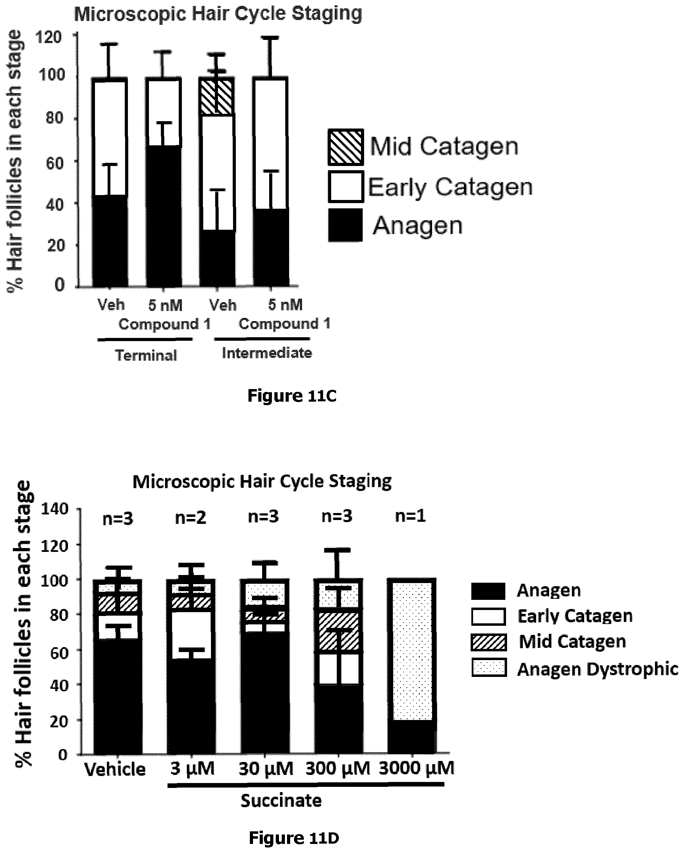

The results are shown in Figures 11A-11D, which show that GPR91 modulation has an impact in the cycling of healthy human hair follicles. Specifically, inhibition of GPR91 increases the percentage of hair follicles in the anagen phase in donors with healthy hair follicles. This can be seen with Compound 1 in Figure 11A and Compound 2 in Figure 11B. In AGA patient donors GPR91 inhibition results in a similar phenotype where there are increased proportion of hair follicles in the anagen phase and there is a prevention of early entry into catagen. This beneficial effect on the hair follicle cycle occurs in the affected region of the scalp in both terminal and intermediate hair follicles. This is demonstrated by Figure 11C. Figure 11D shows that activation of GPR91 decreases the percentage of hair follicles in the anagen phase and stimulates early entry into catagen. At high concentrations, succinate is highly toxic to hair follicles.

### Example 10: GPR91 inhibitors induce versican expression

Hair follicle samples from elective surgeries have been obtained and processed for further analysis using the following method. Studies were conducted according to the Declaration of Helsinki Principles.

Versican immunofluorescence (as previously described in Soma et al J Dermatol Sci 2005, doi: 10.1016/j.jdermsci.2005.03.010) was used to determine hair follicle inductive properties.

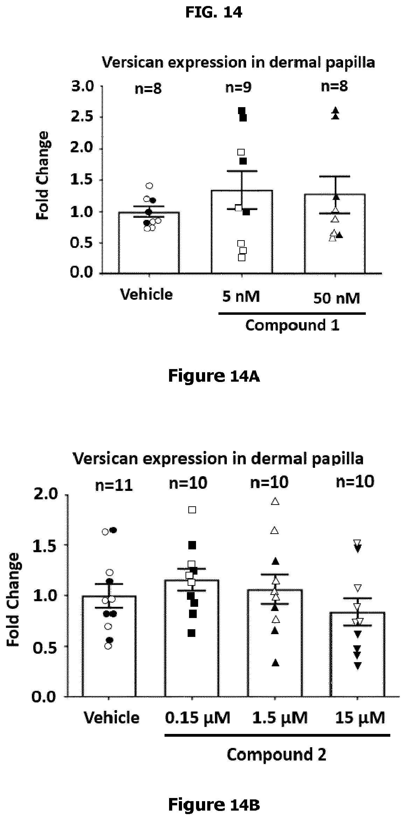

The results shown in Figures 14A, 14B (quantified immunofluorescent signal) and 15A and 15B (representative images), indicate that administration of GPR91 inhibitors increases versican expression. Versican is implicated in the induction of hair morphogenesis, the initiation of hair regeneration, and maintenance of hair growth.

### Example 11: Administration of GPR91 inhibitors increase dermal papilla size

The effect of GPR91 inhibition on dermal papilla size was assessed using the following method.

Hair follicle samples from elective surgeries have been obtained and processed for further analysis using the following method. Studies were conducted according to the Declaration of Helsinki Principles.

Hair follicle organ cultures *ex vivo:* Full length hair follicles were micro dissected from scalp skin or FUEs as described above.

Microscopic hair cycle staging: Hair cycle staging is performed at the end of the culture and can be determined according to several established parameters as described previously. Hair follicles are microscopically evaluated for the hair cycle staging using Masson-Fontana histochemistry and Ki-67/TUNEL immunostaining as previously described. Compounds 1 and 2, at the concentrations indicated, are present throughout the duration of the hair follicle culture at 0.1% DMSO. Melanin clumps are indicated on the images by the orange arrows, and are counted in the Masson-fontana stained section at the end of the culture in a defined region of interest starting at the Auber's line and including the precortical hair matrix as described previously. The change in the area of the dermal papilla area as a result of treatment is quantified by expressing the area of the treated dermal papilla over the vehicle treated dermal papilla, in the same donor, to achieve a fold change.

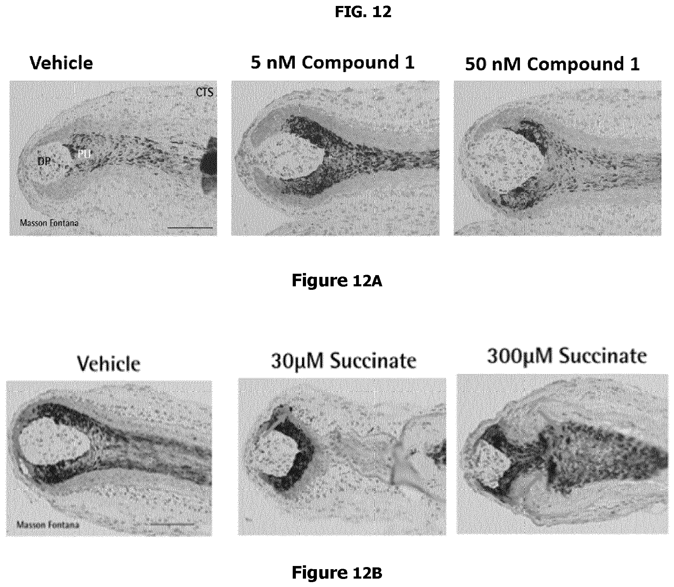

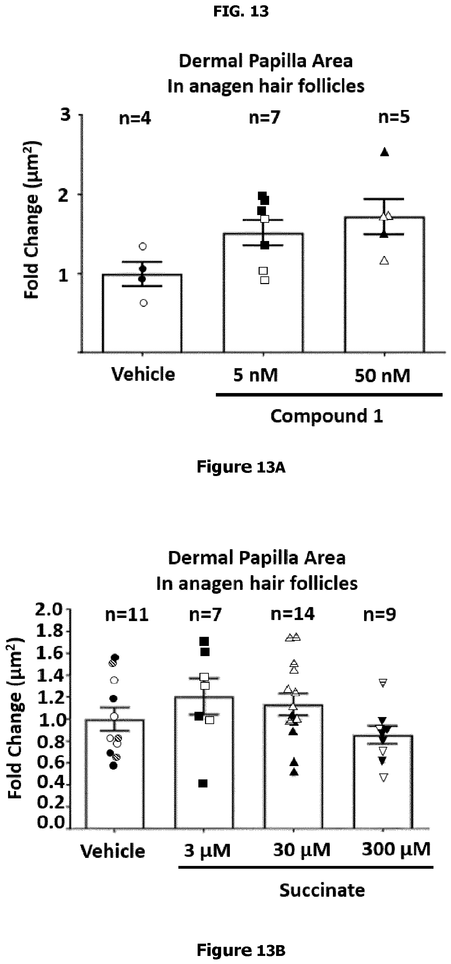

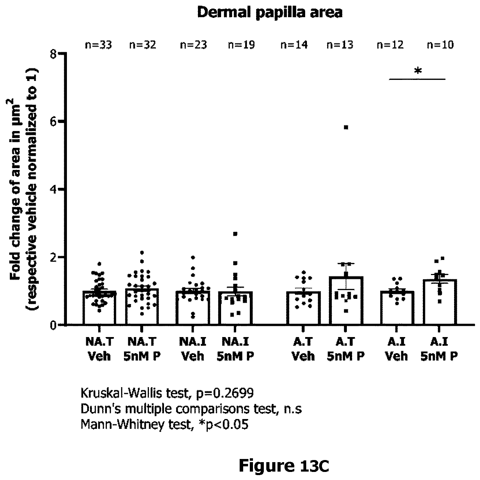

The results are shown in Figures 12A, 12B and 13A-C, which suggest that that GPR91 inhibition produces an increase in the size of the dermal papilla which is predicted to have a disease relevant, positive effect on hair follicle miniaturization. Figures 12A and 12B are representative images of Fontana-Masson stained hair follicles demonstrating the increased size of the dermal papilla observed with GPR91 inhibitors and the decreased size of the dermal papilla and melanin clumping (a marker of toxicity) associated with a GPR91 activator. And Figures 13A and 13B show that GPR91 inhibition increases the dermal papilla size whereas activation decreases the dermal papilla size in healthy hair follicles. Figure 13C shows that in hair follicles from AGA patient donors, there is also an increase in the dermal papilla area with GPR91 inhibition, suggesting a potential reduction in hair follicle miniaturization.

### Example 12: Succinate reduces hair matrix keratinocyte proliferation and may increase apoptosis

The effect of succinate on hair matrix keratinocytes was assessed using the following method.

Hair follicle samples from elective surgeries have been obtained and processed for further analysis using the following method. Studies were conducted according to the Declaration of Helsinki Principles.

Hair follicle organ cultures *ex vivo:* Full length hair follicles were micro dissected from scalp skin or FUEs as above.

Quantitative evaluation of hair matrix keratinocyte proliferation and apoptosis is performed in Ki-67/TUNEL stained sections of the hair follicle. The germinative hair matrix keratinocyte proliferation is calculated as the percentage of KI-67 positive nuclei. Intra-follicular apoptosis is analyzed using TUNEL staining (TdT-mediated dUTP-biotin nick end labelling) as described previously.

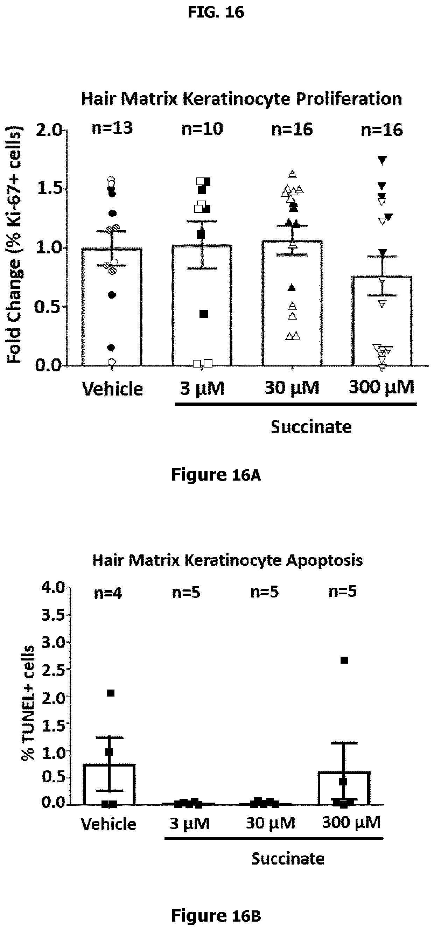

The results are shown in Figures 16A and 16B, and demonstrate that succinate administration reduces hair matrix keratinocyte proliferation and increases apoptosis.

### Example 13: Succinate/GPR91 is a regulator of K15 stem cell proliferation in the bulge basal layer

Hair follicle samples from elective surgeries have been obtained and processed for further analysis using the following method. Studies were conducted according to the Declaration of Helsinki Principles.

Hair follicle organ cultures *ex vivo:* Full length hair follicles were microdissected from scalp skin or FUEs as described above.

Hair follicle bulge stem cells are identified using K15 expression, cellular morphology and anatomical localization. K15 expression is analysed in the bulge and suprabulbar outer root sheath (ORS) of hair follicles. Ki-67 is used as a marker of cell division. The percentage of K15 and Ki67 positive cells is assessed within the bulge region to determine proliferation.

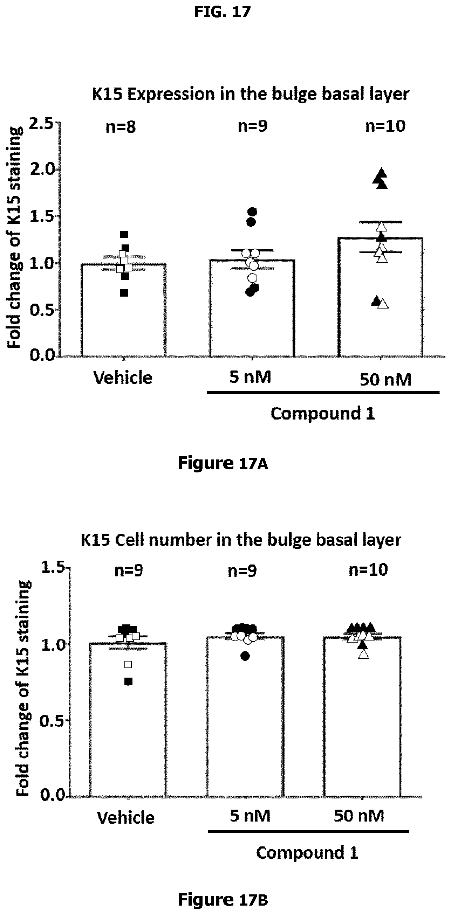

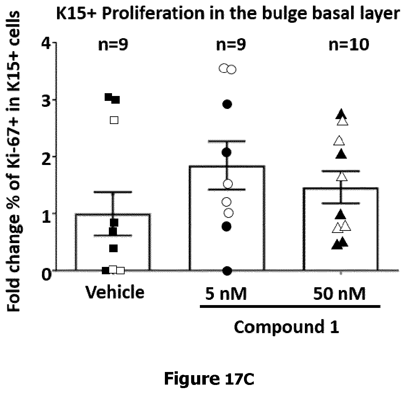

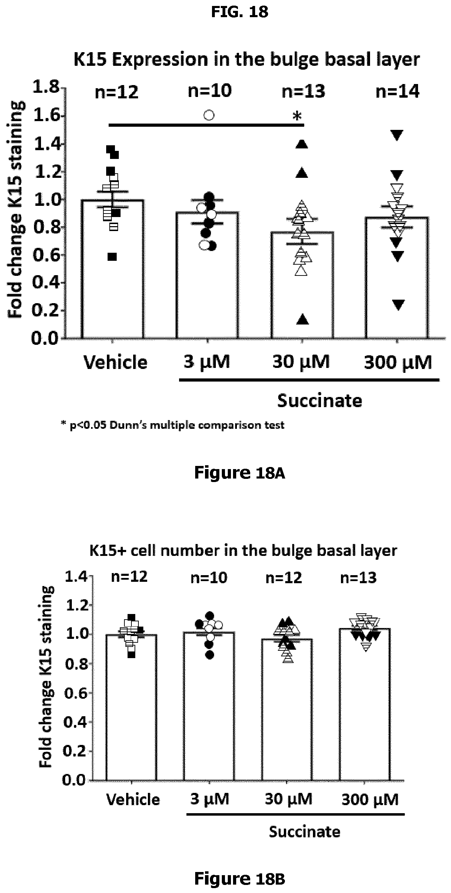

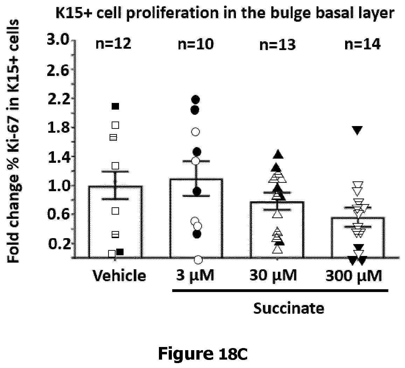

The results are shown in Figures 17A-C and 18A-C. Specifically, Figures 17A to 17C demonstrate that GPR91 inhibition may positively regulate K15 stem cell proliferation in the bulge basal layer, and Figures 18A to 18C demonstrate that GPR91 activation may negatively regulate K15 stem cell proliferation in the bulge basal layer.

### Example 14: RNA sequencing in patient samples identifies succinate related pathways that overlap with androgenetic alopecia and are reversed by GPR91 inhibition.

RNA extraction, sequencing and analysis were performed as follows; RNA from n=2-3 hair follicles per group was extracted using ARCTURUS ^{®} PicoPure ^{®} RNA Isolation Kit (ThermoFisher) including a Dnase digestions step using the Rnase-Free Dnase Set from Qiagen and stored at -80 degrees Celsius until analysed. RNA concentration, purity and integrity were assessed before sequencing. Sequancing was performed on a NovaSeq 6000 using 2x100 bp read length with an output of approximately 30 M clusters (6 Gb per sample). Demultiplexing of the sequencing reads was performed with Illumina bcl2fastq (2.20). Adapters and Pico v2 SMART adapters were trimmed with Skewer (version 0.2.2) (Jiang et al. 2014). Trimmed raw reads were aligned to a GRCh38.105 using STAR (version 2.7.10a) (Dobin et al. 2013). The quality of the FASTQ files was analysed with FastQC (version 0.11.5-cegat) (Andrews 2010).

RNAseq data analysis was performed as follows: Normalized counts were calculated with DESeq2 (version 1.28.1) (Love et al 2014). Briefly, DESeq2 first creates a pseudo-reference sample (row-wise geometric mean) and calculates the ratio of each sample to this reference. Then, the normalization factor for each sample (size factor) is calculated using the median value of all ratios for a given sample. Normalized count values are created by dividing each raw count value in a given sample by that sample's normalization factor. Finally, DESeq2 calculates p values and adjusted p values for genes of two groups by allowing the shrinkage of the log2 fold changes when the information for a gene is low (low counts or high dispersion values). For manual calculation of fold change (FC) between two groups, the ratio of the normalized read counts was used. And FC is considered relevant or significant if it is greater of equal to 2 (upregulation) or smaller that or equal to 0.5 (downregulation).

### Results

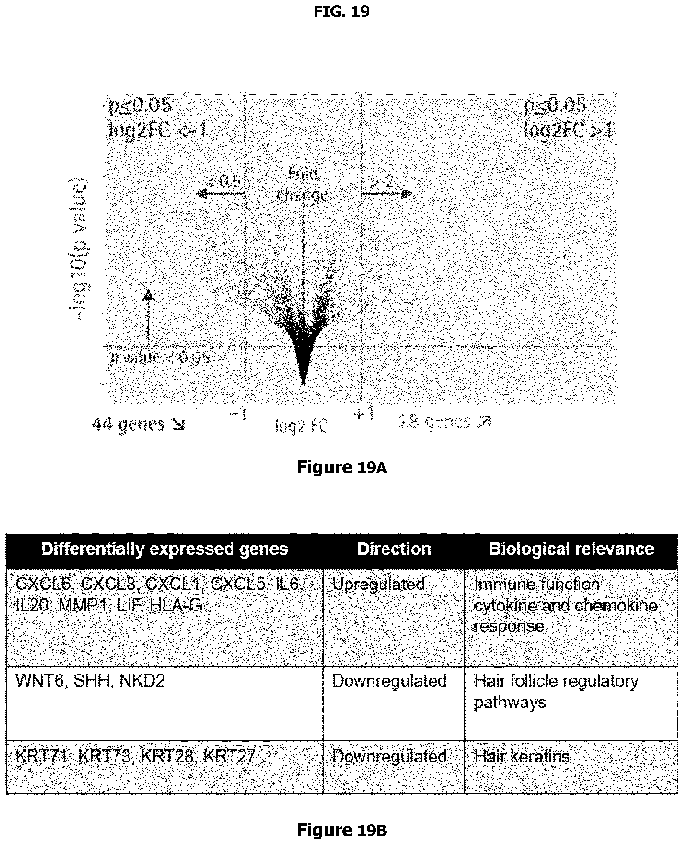

The results are shown in Figure 19 and Figure 20. In healthy hair follicles, treatment with succinate induced an upregulation of 28 genes and downregulation of 44 genes. A volcano plot depicting the spread of differentially regulated genes can be seen in Figure 19A. The upregulated genes correspond to activation of immune function specifically relating to cytokine and chemokine responses which mirror what we observe from stimulation of immune cell in vitro. Down regulated pathways include those that regulate key pathways in hair follicle biology and hair keratins. These would be expected to have a negative impact on hair growth suggesting that at the transcriptional level, succinate induces perifollicular inflammation and inhibition of hair growth. These genes are binned according to their relevant pathways in Figure 19B.

To produce an AGA relevant transcriptional signature, hair follicles were taken from patient donors from either the affected (or balding) regions of the scalp vs the non-affected (or non-balding) regions of the scalp. Comparing these two regions, Figure 20A highlights that 36 genes are upregulated and 34 genes are downregulated in the balding region compared to non-balding. The genes are and associated biological relevant are summarized in Figure 20B. Briefly, genes that would promote infiltration of cells (such as immune cells) are upregulated in balding scalp, which is consistent with the increased number of immune cells shown to be present in these regions. Hair keratins are downregulated and genes that are associated with disruption of the hair follicle cycle and maintenance of telogen are also differentially expressed. This transcriptional profile mirrors the hair follicle cycle dysfunction known to occur in AGA.

Hair follicles from affected regions of the scalp that are treated with GPR91 inhibitors produce a transcriptional profile that is suggestive of a beneficial effect on hair growth, a decrease in apoptotic processes and a reduction in chemotaxis and immune signalling. These genes and associated pathways and biological relevance are summarized in Figure 19C providing evidence at the transcriptional level that GPR91 inhibition may reverse or prevent hair follicle dysfunction associated with AGA.

### Embodiments of the invention

1. An inhibitor of GPR91 for use in the treatment, or prevention, of a hair-loss related disorder or a symptom thereof, in a subject in need thereof.
2. The inhibitor of GPR91 for use according to embodiment 1, wherein the hair-loss related disorder is selected from alopecia areata, alopecia universalis, alopecia totalis, androgenetic alopecia, and telogen effluvium.
3. The inhibitor of GPR91 for use according to embodiments 1 or 2, wherein the hair-loss related disorder is androgenetic alopecia.
4. The inhibitor of GPR91 for use according to embodiments 1-3, wherein the inhibitor of GPR91 comprises a small molecule inhibitor, an antibody or fragment thereof, a binding protein, or an aptamer.
5. The inhibitor of GPR91 for use according to embodiments 1-5, wherein the inhibitor of GPR91 is administered topically.
6. The inhibitor of GPR91 for use according to embodiments 1-5, wherein the inhibitor of GPR91 is administered orally.
7. The inhibitor of GPR91 for use according to any one of embodiments 1-6, wherein the inhibitor of GPR91 is selected from the group consisting of:
   2-(2-(4'-((4-methylpiperazin-1-yl)methyl)-[1,1'-biphenyl]-3-carboxamido)phenyl)acetic acid;
   N-[{3-(3-trifluoromethyl-4-fluorophenyl)isoxazol-5-yl}methyl]-4-([1,8]naphthyridin-2-yl)butyramide;
   2-(2-(4'-chloro-2,2'-difluoro-5'-(piperidin-3-yloxy)-[1,1'-biphenyl]-3-carboxamido)-5-fluorophenyl)acetic acid;
   N-(1-(4'-fluoro-3'-(trifluoromethyl)-[1,1'-biphenyl]-4-yl)ethyl)-4-(1,8-naphthyridin-2-yl)benzamide;
   2-[3-[4-(8-isoquinolyloxy)-2-(trifluoromethyl)phenoxy]phenyl]acetic acid;
   2-[3-[4-phthalazin-5-yloxy-2-(trifluoromethyl)phenoxy]phenyl]acetic acid;
   3-[3-[4-phthalazin-5-yloxy-2-(trifluoromethyl)phenoxy]phenyl]propanoic acid;
   2-[3-[4-(8-chlorophthalazin-5-yl)oxy-2-(trifluoromethyl)phenoxy]phenyl]acetic acid;
   2-[3-[4-(1-methylindazol-7-yl)oxy-2-(trifluoromethyl)phenoxy]phenyl]acetic acid;
   2-[3-[4-(6-quinolyloxy)-2-(trifluoromethyl)phenoxy]phenyl]acetic acid;
   3-[3-[4-imidazo[1,2-a]pyridin-8-yloxy-2-(trifluoromethyl)phenoxy]phenyl]propanoic acid;
   2-[3-[4-[(5-fluoro-8-isoquinolyl)oxy]-2-(trifluoromethyl)phenoxy]phenyl]acetic acid;
   2-[3-[4-quinazolin-5-yloxy-2-(trifluoromethyl)phenoxy]phenyl]acetic acid;
   3-[3-[4-[(2-methyl-3,4-dihydro-1H-isoquinolin-8-yl)oxy]-2-(trifluoromethyl) phenoxy] phenyl]propanoic acid;
   3-[3-[4-(2,3-dihydro-1,4-benzodioxin-5-yloxy)-2-(trifluoromethyl)phenoxy]phenyl]propanoic acid;
   3-[3-[4-(8-quinolyloxy)-2-(trifluoromethyl)phenoxy]phenyl]propanoic acid;
   3-[3-[4-(4-quinolyloxy)-2-(trifluoromethyl)phenoxy]phenyl]propanoic acid;
   3-[3-[4-(5-isoquinolyloxy)-2-(trifluoromethyl)phenoxy]phenyl]propanoic acid;
   2-[3-[4-(2-methylindazol-4-yl)oxy-2-(trifluoromethyl)phenoxy]phenyl]acetic acid;
   2-[3-[4-(1-methylindazol-4-yl)oxy-2-(trifluoromethyl)phenoxy]phenyl]acetic acid;
   2-[3-[4-([1,2,4]triazolo[4,3-a]pyridin-5-yloxy)-2-(trifluoromethyl)phenoxy]phenyl]acetic acid;
   2-[3-[4-(3,4-dihydro-2H-1,4-benzoxazin-6-yloxy)-2-(trifluoromethyl)phenoxy]phenyl]acetic acid;
   2-[3-[4-([1,2,4]triazolo [4,3-a]pyridin-7-yloxy)-2-(trifluoromethyl)phenoxy] phenyl] acetic acid;
   2-[3-[4-[1-(oxetan-3-yl)indol-6-yl]oxy-2-(trifluoromethyl)phenoxy]phenyl]acetic acid;
   2-[3-[4-[1-(oxetan-3-yl)indazol-6-yl]oxy-2-(trifluoromethyl)phenoxy]phenyl]acetic acid;
   2-[3-[4-[2-(oxetan-3-yl)indazol-6-yl]oxy-2-(trifluoromethyl)phenoxy]phenyl]acetic acid;
   2-[3-[4-[1-(oxetan-3-yl)indazol-5-yl]oxy-2-(trifluoromethyl)phenoxy]phenyl]acetic acid;
   2-[3-[4-[2-(oxetan-3-yl)indazol-5-yl]oxy-2-(trifluoromethyl)phenoxy]phenyl]acetic acid;
   2-[3-[4-[3-(oxetan-3-yl)benzotriazol-5-yl]oxy-2-(trifluoromethyl) phenoxy]phenyl]acetic acid;
   2-[3-[4-[3-(oxetan-3-yl)benzimidazol-5-yl]oxy-2-(trifluoromethyl)phenoxy] phenyl]acetic acid;
   2-[3-[4-[2-methyl-3-(oxetan-3-yl)benzimidazol-5-yl]oxy-2-(trifluoromethyl) phenoxy] phenyl]acetic acid;
   2-[3-[2-(difluoromethyl)-4-[2-methyl-3-(oxetan-3-yl)benzimidazol-5-yl]oxy-phenoxy] phenyl]acetic acid;
   2-[3-[4-[1-(oxetan-3-yl)imidazo[4,5-b]pyridin-6-yl]oxy-2-(trifluoromethyl) phenoxy] phenyl]acetic acid;
   2-[3-[4-[1-(oxetan-3-yl)pyrrolo[2,3-b]pyridin-6-yl]oxy-2-(trifluoromethyl) phenoxy] phenyl]acetic acid;
   6-[4-[3-(carboxymethyl)phenoxy]-3-(trifluoromethyl)phenoxy]imidazo[1,5-a]pyridine-3-carboxylic acid;
   2-[3-[4-[1-(carboxymethyl)indol-5-yl]oxy-2-(trifluoromethyl)phenoxy]phenyl]acetic acid;
   2-[3-[4-[1-(carboxymethyl)indol-6-yl]oxy-2-(trifluoromethyl)phenoxy]phenyl]acetic acid;
   3-[4-[4-(8-isoquinolyloxy)-2-(trifluoromethyl)phenoxy]phenyl]propanoic acid;
   3-[3-[4-(2-methylindazol-5-yl)oxy-2-(trifluoromethyl)phenoxy]phenyl]propanoic acid;
   2-[3-[[5-(8-isoquinolyloxy)-3-(trifluoromethyl)-2-pyridyl]oxy]phenyl]acetic acid;
   3-[3-[4-imidazo[1,2-a]pyridin-5-yloxy-2-(trifluoromethyl)phenoxy]phenyl]propanoic acid;
   2-[3-[2-cyclopropyl-4-(8-isoquinolyloxy)phenoxy]phenyl]acetic acid;
   2-[3-[2-cyclopropyl-4-[2-methyl-3-(oxetan-3-yl)benzimidazol-5-yl]oxy-phenoxy] phenyl]acetic acid;
   2-[3-[4-[(1-chloro-8-isoquinolyl)oxy]-2-(trifluoromethyl)phenoxy]phenyl]acetic acid;
   2-[3-[4-[(1-methyl-8-isoquinolyl)oxy]-2-(trifluoromethyl)phenoxy]phenyl]acetic acid;
   3-[3-[4-(8-isoquinolyloxy)-2-(trifluoromethyl)phenoxy]phenyl]propanoic acid;
   3-[3-[4-(1,2,3,4-tetrahydroisoquinolin-8-yloxy)-2-(trifluoromethyl) phenoxy] phenyl] propanoic acid;
   3-[3-[4-indan-1-yloxy-2-(trifluoromethyl)phenoxy]phenyl]propanoic acid;
   3-[3-[4-(1H-indazol-5-yloxy)-2-(trifluoromethyl)phenoxy]phenyl]propanoic acid;
   2-[3-[4-[(3-cyano-1H-indazol-5-yl)oxy]-2-(trifluoromethyl) phenoxy]phenyl]acetic acid;
   5-[4-[3-(carboxymethyl)phenoxy]-3-(trifluoromethyl)phenoxy]-1H-indazole-3-carboxylic acid;
   2-[3-[4-(1H-indazol-4-yloxy)-2-(trifluoromethyl)phenoxy]phenyl]acetic acid;
   2-[3-[4-imidazo[1,5-a]pyridin-5-yloxy-2-(trifluoromethyl)phenoxy]phenyl]acetic acid;
   2-[3-[4-imidazo[1,5-a]pyridin-8-yloxy-2-(trifluoromethyl) phenoxy]phenyl]acetic acid;
   2-[3-[4-(3-methylimidazo[1,5-a]pyridin-5-yl)oxy-2-(trifluoromethyl)phenoxy]phenyl]acetic acid;
   2-[3-[4-(3-methylimidazo[1,5-a]pyridin-8-yl)oxy-2-(trifluoromethyl)phenoxy]phenyl]acetic acid;
   2-[3-[4-[3-(oxetan-3-yl)imidazo[1,5-a]pyridin-6-yl]oxy-2-(trifluoromethyl) phenoxy] phenyl] acetic acid;
   2-[3-[4-(8-isoquinolyloxy)-2-(trifluoromethyl)phenoxy]phenyl]-N-methylsulfonyl-acetamide;
   3-[3-[4-(8-isoquinolyloxy)-2-(trifluoromethyl)phenoxy]phenyl]propanamide;
   2-[3-[4-(8-isoquinolyloxy)-2-(trifluoromethyl)phenoxy]phenyl]acetamide;
   8-[4-[3-[2-(4H-1,2,4-triazol-3-yl)ethyl]phenoxy]-3-(trifluoromethyl)phenoxy]isoquinoline;
   8-[4-[3-[2-(1H-tetrazol-5-yl)ethyl]phenoxy]-3-(trifluoromethyl)phenoxy]isoquinoline;
   8-[4-[3-(1H-tetrazol-5-ylmethyl)phenoxy]-3-(trifluoromethyl) phenoxy]isoquinoline; and
   2-[3-[4-(8-isoquinolyloxy)-3-(trifluoromethyl)phenoxy]phenyl]acetic acid;
   or a pharmaceutically acceptable salt thereof.
8. The inhibitor of GPR91 for use according to any one of embodiments 1-6, wherein the inhibitor of GPR91 is selected from the group consisting of:
   2-(2-(4'-((4-methylpiperazin-1-yl)methyl)-[1,1'-biphenyl]-3-carboxamido)phenyl)acetic acid having a structure represented by 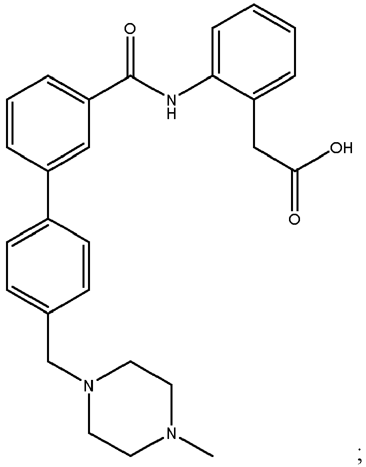
   N-[{3-(3-trifluoromethyl-4-fluorophenyl)isoxazol-5-yl}methyl]-4-([1,8]naphthyridin-2-yl)butyramide) having a structure represented by 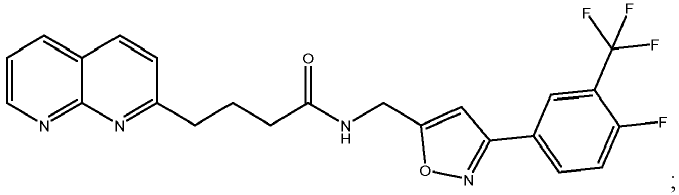 or
   a pharmaceutically acceptable salt thereof.
9. The inhibitor of GPR91 for use according to any one of embodiments 1-6, wherein the use further comprises administration of an effective amount of an androgen receptor antagonist, JAK inhibitor, minoxidil, finasteride, dutasteride, antiandrogens, hair transplantation procedures, prostaglandin analogues, light therapy, platelet-rich plasma, or microneedling.
10. The inhibitor of GPR91 according to any one of embodiments 19, wherein the subject has a decreased number of hair follicles in the anagen phase relative to a control subject prior to administering a therapeutically effective amount of a GPR91 inhibitor.
11. The inhibitor of GPR91 according to any one of embodiments 1-10, wherein the subject is afflicted with, or is at risk of developing, alopecia from an underlying health disorder or genetic disposition.
12. A method for treating or preventing a hair-loss related disorder or a symptom thereof in a subject in need thereof comprising administering to a human a therapeutically effective amount of an inhibitor of human GPR91.
13. The method according to embodiment 12, wherein the hair-loss related disorder is selected from alopecia areata, alopecia universalis, alopecia totalis, androgenetic alopecia, and telogen effluvium.
14. The method according to embodiment 12, wherein the hair-loss related disorder is androgenetic alopecia.
15. The method according to embodiment 12, wherein the inhibitor of GPR91 comprises a small molecule inhibitor, an antibody or fragment thereof, a binding protein, or an aptamer.
16. The method according to embodiment 12 or 13, wherein the inhibitor of GPR91 is administered topically.
17. The method according to embodiment 12 or 13, wherein the inhibitor of GPR91 is administered orally.
18. The method according to any one of embodiments 12 to 15, wherein the inhibitor of GPR91 is selected from the group consisting of:
   2-(2-(4'-((4-methylpiperazin-1-yl)methyl)-[1,1'-biphenyl]-3-carboxamido)phenyl)acetic acid;
   N-[{3-(3-trifluoromethyl-4-fluorophenyl)isoxazol-5-yl}methyl]-4-([1,8]naphthyridin-2-yl)butyramide;
   2-(2-(4'-chloro-2,2'-difluoro-5'-(piperidin-3-yloxy)-[1,1'-biphenyl]-3-carboxamido)-5-fluorophenyl)acetic acid;
   N-(1-(4'-fluoro-3'-(trifluoromethyl)-[1,1'-biphenyl]-4-yl)ethyl)-4-(1,8-naphthyridin-2-yl)benzamide;
   2-[3-[4-(8-isoquinolyloxy)-2-(trifluoromethyl)phenoxy]phenyl]acetic acid;
   2-[3-[4-phthalazin-5-yloxy-2-(trifluoromethyl)phenoxy]phenyl]acetic acid;
   3-[3-[4-phthalazin-5-yloxy-2-(trifluoromethyl)phenoxy]phenyl]propanoic acid;
   2-[3-[4-(8-chlorophthalazin-5-yl)oxy-2-(trifluoromethyl)phenoxy]phenyl]acetic acid;
   2-[3-[4-(1-methylindazol-7-yl)oxy-2-(trifluoromethyl)phenoxy]phenyl]acetic acid;
   2-[3-[4-(6-quinolyloxy)-2-(trifluoromethyl)phenoxy]phenyl]acetic acid;
   3-[3-[4-imidazo[1,2-a]pyridin-8-yloxy-2-(trifluoromethyl)phenoxy]phenyl]propanoic acid;
   2-[3-[4-[(5-fluoro-8-isoquinolyl)oxy]-2-(trifluoromethyl)phenoxy]phenyl]acetic acid;
   2-[3-[4-quinazolin-5-yloxy-2-(trifluoromethyl)phenoxy]phenyl]acetic acid;
   3-[3-[4-[(2-methyl-3,4-dihydro-1H-isoquinolin-8-yl)oxy]-2-(trifluoromethyl) phenoxy] phenyl]propanoic acid;
   3-[3-[4-(2,3-dihydro-1,4-benzodioxin-5-yloxy)-2-(trifluoromethyl)phenoxy]phenyl]propanoic acid;
   3-[3-[4-(8-quinolyloxy)-2-(trifluoromethyl)phenoxy]phenyl]propanoic acid;
   3-[3-[4-(4-quinolyloxy)-2-(trifluoromethyl)phenoxy]phenyl]propanoic acid;
   3-[3-[4-(5-isoquinolyloxy)-2-(trifluoromethyl)phenoxy]phenyl]propanoic acid;
   2-[3-[4-(2-methylindazol-4-yl)oxy-2-(trifluoromethyl)phenoxy]phenyl]acetic acid;
   2-[3-[4-(1-methylindazol-4-yl)oxy-2-(trifluoromethyl)phenoxy]phenyl]acetic acid;
   2-[3-[4-([1,2,4]triazolo[4,3-a]pyridin-5-yloxy)-2-(trifluoromethyl)phenoxy]phenyl]acetic acid;
   2-[3-[4-(3,4-dihydro-2H-1,4-benzoxazin-6-yloxy)-2-(trifluoromethyl)phenoxy]phenyl]acetic acid;
   2-[3-[4-([1,2,4]triazolo [4,3-a]pyridin-7-yloxy)-2-(trifluoromethyl)phenoxy] phenyl] acetic acid;
   2-[3-[4-[1-(oxetan-3-yl)indol-6-yl]oxy-2-(trifluoromethyl)phenoxy]phenyl]acetic acid;
   2-[3-[4-[1-(oxetan-3-yl)indazol-6-yl]oxy-2-(trifluoromethyl)phenoxy]phenyl]acetic acid;
   2-[3-[4-[2-(oxetan-3-yl)indazol-6-yl]oxy-2-(trifluoromethyl)phenoxy]phenyl]acetic acid;
   2-[3-[4-[1-(oxetan-3-yl)indazol-5-yl]oxy-2-(trifluoromethyl)phenoxy]phenyl]acetic acid;
   2-[3-[4-[2-(oxetan-3-yl)indazol-5-yl]oxy-2-(trifluoromethyl)phenoxy]phenyl]acetic acid;
   2-[3-[4-[3-(oxetan-3-yl)benzotriazol-5-yl]oxy-2-(trifluoromethyl) phenoxy]phenyl]acetic acid;
   2-[3-[4-[3-(oxetan-3-yl)benzimidazol-5-yl]oxy-2-(trifluoromethyl)phenoxy] phenyl]acetic acid;
   2-[3-[4-[2-methyl-3-(oxetan-3-yl)benzimidazol-5-yl]oxy-2-(trifluoromethyl) phenoxy] phenyl]acetic acid;
   2-[3-[2-(difluoromethyl)-4-[2-methyl-3-(oxetan-3-yl)benzimidazol-5-yl]oxy-phenoxy] phenyl]acetic acid;
   2-[3-[4-[1-(oxetan-3-yl)imidazo[4,5-b]pyridin-6-yl]oxy-2-(trifluoromethyl) phenoxy] phenyl]acetic acid;
   2-[3-[4-[1-(oxetan-3-yl)pyrrolo[2,3-b]pyridin-6-yl]oxy-2-(trifluoromethyl) phenoxy] phenyl]acetic acid;
   6-[4-[3-(carboxymethyl)phenoxy]-3-(trifluoromethyl)phenoxy]imidazo[1,5-a]pyridine-3-carboxylic acid;
   2-[3-[4-[1-(carboxymethyl)indol-5-yl]oxy-2-(trifluoromethyl)phenoxy]phenyl]acetic acid;
   2-[3-[4-[1-(carboxymethyl)indol-6-yl]oxy-2-(trifluoromethyl)phenoxy]phenyl]acetic acid;
   3-[4-[4-(8-isoquinolyloxy)-2-(trifluoromethyl)phenoxy]phenyl]propanoic acid;
   3-[3-[4-(2-methylindazol-5-yl)oxy-2-(trifluoromethyl)phenoxy]phenyl]propanoic acid;
   2-[3-[[5-(8-isoquinolyloxy)-3-(trifluoromethyl)-2-pyridyl]oxy]phenyl]acetic acid;
   3-[3-[4-imidazo[1,2-a]pyridin-5-yloxy-2-(trifluoromethyl)phenoxy]phenyl]propanoic acid;
   2-[3-[2-cyclopropyl-4-(8-isoquinolyloxy)phenoxy]phenyl]acetic acid;
   2-[3-[2-cyclopropyl-4-[2-methyl-3-(oxetan-3-yl)benzimidazol-5-yl]oxy-phenoxy] phenyl]acetic acid;
   2-[3-[4-[(1-chloro-8-isoquinolyl)oxy]-2-(trifluoromethyl)phenoxy]phenyl]acetic acid;
   2-[3-[4-[(1-methyl-8-isoquinolyl)oxy]-2-(trifluoromethyl)phenoxy]phenyl]acetic acid;
   3-[3-[4-(8-isoquinolyloxy)-2-(trifluoromethyl)phenoxy]phenyl]propanoic acid;
   3-[3-[4-(1,2,3,4-tetrahydroisoquinolin-8-yloxy)-2-(trifluoromethyl) phenoxy] phenyl] propanoic acid;
   3-[3-[4-indan-1-yloxy-2-(trifluoromethyl)phenoxy]phenyl]propanoic acid;
   3-[3-[4-(1H-indazol-5-yloxy)-2-(trifluoromethyl)phenoxy]phenyl]propanoic acid;
   2-[3-[4-[(3-cyano-1H-indazol-5-yl)oxy]-2-(trifluoromethyl) phenoxy]phenyl]acetic acid;
   5-[4-[3-(carboxymethyl)phenoxy]-3-(trifluoromethyl)phenoxy]-1H-indazole-3-carboxylic acid;
   2-[3-[4-(1H-indazol-4-yloxy)-2-(trifluoromethyl)phenoxy]phenyl]acetic acid;
   2-[3-[4-imidazo[1,5-a]pyridin-5-yloxy-2-(trifluoromethyl)phenoxy]phenyl]acetic acid;
   2-[3-[4-imidazo[1,5-a]pyridin-8-yloxy-2-(trifluoromethyl) phenoxy]phenyl]acetic acid;
   2-[3-[4-(3-methylimidazo[1,5-a]pyridin-5-yl)oxy-2-(trifluoromethyl)phenoxy]phenyl]acetic acid;
   2-[3-[4-(3-methylimidazo[1,5-a]pyridin-8-yl)oxy-2-(trifluoromethyl)phenoxy]phenyl]acetic acid;
   2-[3-[4-[3-(oxetan-3-yl)imidazo[1,5-a]pyridin-6-yl]oxy-2-(trifluoromethyl) phenoxy] phenyl] acetic acid;
   2-[3-[4-(8-isoquinolyloxy)-2-(trifluoromethyl)phenoxy]phenyl]-N-methylsulfonyl-acetamide;
   3-[3-[4-(8-isoquinolyloxy)-2-(trifluoromethyl)phenoxy]phenyl]propanamide;
   2-[3-[4-(8-isoquinolyloxy)-2-(trifluoromethyl)phenoxy]phenyl]acetamide;
   8-[4-[3-[2-(4H-1,2,4-triazol-3-yl)ethyl]phenoxy]-3-(trifluoromethyl)phenoxy]isoquinoline;
   8-[4-[3-[2-(1H-tetrazol-5-yl)ethyl]phenoxy]-3-(trifluoromethyl)phenoxy]isoquinoline;
   8-[4-[3-(1H-tetrazol-5-ylmethyl)phenoxy]-3-(trifluoromethyl) phenoxy]isoquinoline; and
   2-[3-[4-(8-isoquinolyloxy)-3-(trifluoromethyl)phenoxy]phenyl]acetic acid;
   or a pharmaceutically acceptable salt thereof.
19. The method according to any one of embodiments 10 to 14, wherein the inhibitor of GPR91 is selected from the group consisting of:
   2-(2-(4'-((4-methylpiperazin-1-yl)methyl)-[1,1'-biphenyl]-3-carboxamido)phenyl)acetic acid having a structure represented by 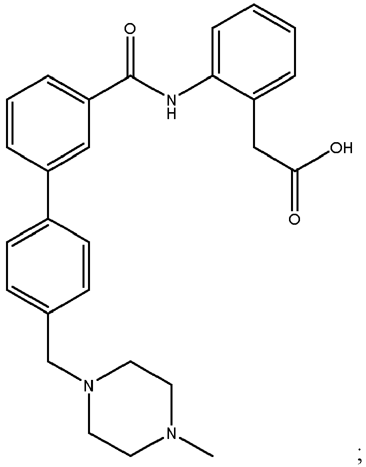 and
   N-[{3-(3-trifluoromethyl-4-fluorophenyl)isoxazol-5-yl}methyl]-4-([1,8]naphthyridin-2-yl)butyramide) having a structure represented by 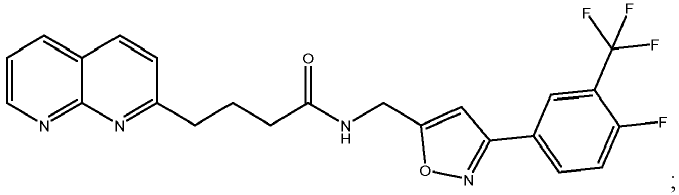 or
   a pharmaceutically acceptable salt thereof.
20. The method according to any one of embodiments 12 to 19, wherein the method further comprises administration of an effective amount of androgen receptor antagonist, JAK inhibitor, minoxidil, finasteride, dutasteride, antiandrogens, hair transplantation procedures, prostaglandin analogues, light therapy, platelet-rich plasma, or microneedling.
21. The method according to any one of embodiments 12 to 20, wherein the subject has a decreased number of hair follicles in the anagen phase relative to a control subject prior to administering a therapeutically effective amount of a GPR91 inhibitor.
22. The method according to any one of embodiments 12 to 21, wherein the subject is afflicted with, or is at risk of developing, alopecia from an underlying health disorder or genetic disposition.
23. Use of an inhibitor of GPR91 in the manufacture of a medicament for use in the treatment or prevention of a hair-loss related disorder or a symptom thereof in a subject in need thereof.
24. The use according to embodiment 23, wherein the hair-loss related disorder is selected from alopecia areata, alopecia universalis, alopecia totalis, androgenetic alopecia, and telogen effluvium.
25. The use according to embodiment 23, wherein the hair-loss related disorder is androgenetic alopecia.
26. An inhibitor of GPR91 for use in inducing, or promoting, hair growth in a human.
27. A method of inducing, or promoting, hair growth in a human, the method comprising administering an effective amount of an inhibitor of GPR91.
28. The inhibitor of GPR91 for use according to embodiment 26, or method according to embodiment 27, wherein inducing hair growth induces a transition from a telogen phase to an anagen phase of a hair growth cycle.
29. The inhibitor of GPR91 for use according to embodiment 26, or method according to embodiment 27, wherein the inhibitor of GPR91 increases a number of hair follicles present in the subject in the anagen phase.

REFERENCE SEQUENCE LISTINGS
SEQ ID No. 1 - NM_033050.6 Homo sapiens succinate receptor 1 (SUCNR1), mRNA
GTCAGAAGCTATATCCAGCTGCTGGCAGAGTTCCTGTCAAGGGATCAAGTCTT
CCAACAGAATGGTTATGGTTTAACTCAGCAGAATTTGTTGAACAACTACGACA
TGCTGGGGATCATGGCATGGAATGCAACTTGCAAAAACTGGCTGGCAGCAGAG
GCTGCCCTGGAAAAGTACTACCTTTCCATTTTTTATGGGATTGAGTTCGTTGTG
GGAGTCCTTGGAAATACCATTGTTGTTTACGGCTACATCTTCTCTCTGAAGAAC
TGGAACAGCAGTAATATTTATCTCTTTAACCTCTCTGTCTCTGACTTAGCTTTTC
TGTGCACCCTCCCCATGCTGATAAGGAGTTATGCCAATGGAAACTGGATATAT
GGAGACGTGCTCTGCATAAGCAACCGATATGTGCTTCATGCCAACCTCTATAC
CAGCATTCTCTTTCTCACTTTTATCAGCATAGATCGATACTTGATAATTAAGTA
TCCTTTCCGAGAACACCTTCTGCAAAAGAAAGAGTTTGCTATTTTAATCTCCTT
GGCCATTTGGGTTTTAGTAACCTTAGAGTTACTACCCATACTTCCCCTTATAAA
TCCTGTTATAACTGACAATGGCACCACCTGTAATGATTTTGCAAGTTCTGGAGA
CCCCAACTACAACCTCATTTACAGCATGTGTCTAACACTGTTGGGGTTCCTTAT
TCCTCTTTTTGTGATGTGTTTCTTTTATTACAAGATTGCTCTCTTCCTAAAGCAG
AGGAATAGGCAGGTTGCTACTGCTCTGCCCCTTGAAAAGCCTCTCAACTTGGTC
ATCATGGCAGTGGTAATCTTCTCTGTGCTTTTTACACCCTATCACGTCATGCGG
AATGTGAGGATCGCTTCACGCCTGGGGAGTTGGAAGCAGTATCAGTGCACTCA
GGTCGTCATCAACTCCTTTTACATTGTGACACGGCCTTTGGCCTTTCTGAACAG
TGTCATCAACCCTGTCTTCTATTTTCTTTTGGGAGATCACTTCAGGGACATGCT
GATGAATCAACTGAGACACAACTTCAAATCCCTTACATCCTTTAGCAGATGGG
CTCATGAACTCCTACTTTCATTCAGAGAAAAGTGAGGGGCTTGTGAAACAGAT
TGTTCTACAGATGAATCTGTAAGCCAGTTACAGTTTGCCTTAACTCATAGACAT
CAATCAGAGAGTGTCACAGATTTAACCTTGATCTAAAGACAAGTTGTACCCAG
AGTATGTGAAAAGAATGGGACGACAAGAATGTACTGGTTTCTTCCTCTAAGAA
TTGAAAGGAGTTGAACTGCCTTATGTTTGGGCATGTAACTCCAAAATACTAGG
TAGTATAAGGCTTTCTCAATCAGTGCAAAAATGGAAGATATATAAAGCAACAA
GTTGTCTGCATTTGATCACTGGTCAGATTGTAAAAAAAAAAAAAATTGTTTTGG
CAACATTCTCTATGTTATTCTTATTCACATGATCCTAGAACTTTATGTGAGAAC
TGAATAGCAGAAGATATATTATTAAATAGTTTTTGGAAGTTGTGCTGTTAAGCA
AATGTAAAGTCAACAGTAACAATGATTAAGAAAGGAATATAATGAGTTCAAA

GATATCTATCTGATATATTTTTTATATTCATCATGAATCGGGGTAGTCTTGTAAT
TTATCTAACCTTTTTGTTGTATGGGTTTATAGTGGAAAGAATCTTAATTTAAAT
AAAAAGAACCTGAAGAGATAATTCAACAAAAGTAGAGAATATGTTTGAGCTT
GTCAGAGAATTTAAGTATCAAAATACTAGAGAGATACACCAAGGATATTTGAA
TAAAAAAAAAATTTGTAGAACACAAAATGCCTATCAGGAAACCCAAAGCAAA
ACAGAACAAAAAGTGAATACATCGTTTTAAATGATGATTTTGCCATCTGGTATT
TTATTATCATAATTTGACCACCAGTGCATGAAAATATATTAAAGTTCATTAGCC
TTCTGCTCTTGATTTAATAGTGAACTTTAAAATATATTAATATCATAAACCAGC
AAATAAGGAGAAATAAACTGATAATACTAGATTCATTGGGATAGTTTTCCCTT
TTGGAGGGAAAGTCACAATTTTTCTCCATGATCCCCCATTTTTTGTAAACACTG
GAATAATTTACAAGGATAACTTTATTGTAGCGAGAGCAAATCACATCTGGAGT
GAGCCTTGTTTTCATGCAACATTACCATAAGACCATTGGAGTAAATGTTTCAAA
CAAAATGTACTTCATGAAATTATAGAACATAAAAATATTTATGTGCATATTAA
CCTGTATTATCCCAGGAGGGTTAATGAGGCCCCATTTCCTGCACAAAACAAAT
GTAGGGGAAAAAATGGGTGCTGCATTAGCTTTTTAAAGTCAAACAACTATCAC
ATAATAATTCCTTGATATGCAACATAAAGTACCTATGGCCACATATCTAAGTTC
CTTTCCTTGATGTCATTAGCTATAAGGAGTGAGTAATGTGAAGGACAAAATAA
GCATTATGAATACAGAGACATAAAATTCCACTGTCCCTTCCTTTTCTGAAATAC
TCTGGGAGGTATTCTGATATTTTTAAATCCCCCAATTTTCCCAGTTGATATCTCT
AGACTTCAAGCAACTGAAATATTATAAAAATGCATTAGTCACAAAAGATTTAT
TTTCTTACATCACACCAGGCTTTAAGATATTTTCTGCCATGCCTTGCCTTTAGTA
TCAATATTTTAAAAATTTCACTGTAATTTTATAACTGCCTAATAACAAACTGAA
AACAGAACTTCTGAGTTTCCTTATCAGAAAGGGCCACAGAAAAGGCCTTAAGA
AAGAAAGATTCACTTACTTCTGCAAACTTGGCAATCCGTAATTTAACTTTAATT
TGTATTTTATGCATGAATTTTACTTGTAAAGACAAAATTAAATTTGCTTCCCCT
ACATCCCAACTCCAAGAAGTCACAGGAATAGACTCTTTCTAATAGTGCAGTGA
AATATTTTTTCAAACCATAAACTCACATATATATATATATATATATATATATAT
ATATATATATATGTACCTTGTAAACAAGAGGTTAAGAAATTTTATAGCACATTT
TAAAATTATTTTCTTCCTCAAATCTATCATGTGGTAACTTAATCTTTAACAGACT
TTAGGGCCCTAAACTAAAATTATAAGAGCTATTTCATTTGCTTGTTGTACTGGG
TAATTGTAAGAATCACAAACAATAAATCATGAAAATCCTTTCTGGGTTTGTGGT
TTCTAAAGGAAATAATATTGAGCTTTTGTATTTTGTCTTTTAATTGGCTCCCTGG
TATCTTCAGAATGATGTCTAGATTCTTCAGTCTGGACCCACCTACTCTTCTCAC
ATTATCTCCCATCACTGTTGTCACAAACCATACCCTATAGCTAAAGATTGCTAC

TTTAGTTTATTACATTCAGAACTTTCTTCACTTTGTTCACCTGTTGCCCATCTTT
GTTCTATCATACCTGAATGGTATAATAATTCTAAACAACTTTCAAGGTTCATCT
CATCTGTCATTCTTTGTTGAAGCTTGCCCTTATATCCTCTCTTCCCCAAACCTAA
AAGTAAAACTTTCTACCACTGGGGCTTCAGATACTTTTAATACTACTTATTTGA
ATTTCATTATTTTTATTATAGTTTATTATTGTCTTCATAGCTGAGTACTCTTAAA
ATTACTGTTTATTATAAGTGAATGTCTTACAGAACAAACTCAAAGTCAATGCTT
CTTTTGCATGTGAATAATCAAGCTATTTTTTTGTCATCATTACTTTAGTAAGTAA
CATTATTTTATATGAAGAATAGAGTGTATATACATAAACCCATGTGCCAGCTGC
CATTGCACTGCCTCACATTTTCCTTTAGATGTTTGAGCATAGTTTTAATGACTGT
TTGAAATCTCTGTCTCCTAGTTCCAAATATGGGTCATCTTGGAGTTGGTCTTCA
TTGGTGCCTTTTCTCTTCAGTATGAGTCATAATTTTCCTGTTTCTCTATATGCCT
AGTAAATTAGAATTTTATTCTAGATGATGAATAATGTGTGGCAGAGATTCTGG
ATTCTGTTACATTCCATCAAAGAGTATTGATATTTTGTATATACATTTACAAAT
GTACATGTACACAATAATAAAAATAATTTTTTTCTCAGA

SEQ ID No. 2- Amino Acid (protein sequence) NP_149039.2 succinate receptor 1 [Homo sapiens]:
MLGIMAWNATCKNWLAAEAALEKYYLSIFYGIEFVVGVLGNTIVVYGYIF
SLKNWNSSNIYLFNLSVSDLAFLCTLPMLIRSYANGNWIYGDVLCISNRYVLHANL
YTSILFLTFISIDRYLIIKYPFREHLLQKKEFAILISLAIWVLVTLELLPILPLINPVITDN
GTTCNDFASSGDPNYNLIYSMCLTLLGFLIPLFVMCFFYYKIALFLKQRNRQVATA
LPLEKPLNLVIMAVVIFSVLFTPYHVMRNVRIASRLGSWKQYQCTQVVINSFYIVT
RPLAFLNSVINPVFYFLLGDHFRDMLMNQLRHNFKSLTSFSRWAHELLLSFREK

SEQ ID NO. 3:
<219> 2
<211> 330
<212> PRT
<213> Homo sapiens
<400> 2
Met Ala Trp Asn Ala Thr Cys Lys Asn Trp Leu Ala Ala Glu Ala Ala
1               5                   10                  15

Leu Glu Lys Tyr Tyr Leu Ser Ile Phe Tyr Gly Ile Glu Phe Val Val
            20                  25                  30
Gly Val Leu Gly Asn Thr Ile Val Val Tyr Gly Tyr Ile Phe Ser Leu
        35                  40                  45
Lys Asn Trp Asn Ser Ser Asn Ile Tyr Leu Phe Asn Leu Ser Val Ser
    50                  55                  60
Asp Leu Ala Phe Leu Cys Thr Leu Pro Met Leu Ile Arg Ser Tyr Ala
65                  70                  75                  80
Asn Gly Asn Trp Ile Tyr Gly Asp Val Leu Cys Ile ser Asn Arg Tyr
                85                  90                  95
Val Leu His Ala Asn Leu Tyr Thr Ser Ile Leu Phe Leu Thr Phe Ile
            100                 105                 110
Ser Ile Asp Arg Tyr Leu Ile Ile Lys Tyr Pro Phe Arg Glu His Leu
        115                 120                 125
Leu Gln Lys Lys Glu Phe Ala Ile Leu Ile Ser Leu Ala Ile Trp Val
    130                 135                 140
Leu Val Thr Leu Glu Leu Leu Pro Ile Leu Pro Leu Ile Asn Pro Val
145                 150                 155                 160
Ile Thr Asp Asn Gly Thr Thr Cys Asn Asp Phe Ala Ser Ser Gly Asp
                165                 170                 175
Pro Asn Tyr Asn Leu Ile Tyr Ser Met Cys Leu Thr Leu Leu Gly Phe
            180                 185                 190
Leu Ile Pro Leu Phe Val Met Cys Phe Phe Tyr Tyr Lys Ile Ala Leu
        195                 200                 205
Phe Leu Lys Gln Arg Asn Arg Gln Val Ala Thr Ala Leu Pro Leu Glu
    210                 215                 220
Lys Pro Leu Asn Leu Val Ile Met Ala Val Val Ile Phe Ser Val Leu
225                 230                 235                 240
Phe Thr Pro Tyr His Val Met Arg Asn Val Arg Ile Ala Ser Arg Leu
                245                 250                 255
Gly Ser Trp Lys Gln Tyr Gln Cys Thr Gln Val Val Ile Asn Ser Phe

            260                 265                 270
Tyr Ile Val Thr Arg Pro Leu Ala Phe Leu Asn Ser Val Ile Asn Pro
        275                 280                 285
Val Phe Tyr Phe Leu Leu Gly Asp His Phe Arg Asp Met Leu Met Asn
    290                 295                 300
Gln Leu Arg His Asn Phe Lys Ser Leu Thr Ser Phe Ser Arg Trp Ala
305                 310                 315                 320
His Glu Leu Leu Leu Ser Phe Arg Glu Lys
                325                 330

## Claims

1. An inhibitor of GPR91 for use in the treatment, or prevention, of a hair-loss related disorder or a symptom thereof, in a subject in need thereof.

2. The inhibitor of GPR91 for use according to Claim 1, wherein the hair-loss related disorder is selected from alopecia areata, alopecia universalis, alopecia totalis, androgenetic alopecia, and telogen effluvium.

3. The inhibitor of GPR91 for use according to Claim 1 or 2, wherein the hair-loss related disorder is androgenetic alopecia.

4. The inhibitor of GPR91 for use according to Claim 1-3, wherein the inhibitor of GPR91 comprises a small molecule inhibitor, an antibody or fragment thereof, a binding protein, or an aptamer.

5. The inhibitor of GPR91 for use according to Claim 1-5, wherein the inhibitor of GPR91 is administered topically.

6. The inhibitor of GPR91 for use according to Claim 1-5, wherein the inhibitor of GPR91 is administered orally.

7. The inhibitor of GPR91 for use according to any one of Claims 1-6, wherein the inhibitor of GPR91 is selected from the group consisting of:
2-(2-(4'-((4-methylpiperazin-1-yl)methyl)-[1,1'-biphenyl]-3-carboxamido)phenyl)acetic acid;
N-[{3-(3-trifluoromethyl-4-fluorophenyl)isoxazol-5-yl}methyl]-4-([1,8]naphthyridin-2-yl)butyramide;
2-(2-(4'-chloro-2,2'-difluoro-5'-(piperidin-3-yloxy)-[1,1'-biphenyl]-3-carboxamido)-5-fluorophenyl)acetic acid;
N-(1-(4'-fluoro-3'-(trifluoromethyl)-[1,1'-biphenyl]-4-yl)ethyl)-4-(1,8-naphthyridin-2-yl)benzamide;
2-[3-[4-(8-isoquinolyloxy)-2-(trifluoromethyl)phenoxy]phenyl]acetic acid;
2-[3-[4-phthalazin-5-yloxy-2-(trifluoromethyl)phenoxy]phenyl]acetic acid;
3-[3-[4-phthalazin-5-yloxy-2-(trifluoromethyl)phenoxy]phenyl]propanoic acid;
2-[3-[4-(8-chlorophthalazin-5-yl)oxy-2-(trifluoromethyl)phenoxy]phenyl]acetic acid;
2-[3-[4-(1-methylindazol-7-yl)oxy-2-(trifluoromethyl)phenoxy]phenyl]acetic acid;
2-[3-[4-(6-quinolyloxy)-2-(trifluoromethyl)phenoxy]phenyl]acetic acid;
3-[3-[4-imidazo[1,2-a]pyridin-8-yloxy-2-(trifluoromethyl)phenoxy]phenyl]propanoic acid;
2-[3-[4-[(5-fluoro-8-isoquinolyl)oxy]-2-(trifluoromethyl)phenoxy]phenyl]acetic acid;
2-[3-[4-quinazolin-5-yloxy-2-(trifluoromethyl)phenoxy]phenyl]acetic acid;
3-[3-[4-[(2-methyl-3,4-dihydro-1H-isoquinolin-8-yl)oxy]-2-(trifluoromethyl) phenoxy] phenyl]propanoic acid;
3-[3-[4-(2,3-dihydro-1,4-benzodioxin-5-yloxy)-2-(trifluoromethyl)phenoxy]phenyl]propanoic acid;
3-[3-[4-(8-quinolyloxy)-2-(trifluoromethyl)phenoxy]phenyl]propanoic acid;
3-[3-[4-(4-quinolyloxy)-2-(trifluoromethyl)phenoxy]phenyl]propanoic acid;
3-[3-[4-(5-isoquinolyloxy)-2-(trifluoromethyl)phenoxy]phenyl]propanoic acid;
2-[3-[4-(2-methylindazol-4-yl)oxy-2-(trifluoromethyl)phenoxy]phenyl]acetic acid;
2-[3-[4-(1-methylindazol-4-yl)oxy-2-(trifluoromethyl)phenoxy]phenyl]acetic acid;
2-[3-[4-([1,2,4]triazolo[4,3-a]pyridin-5-yloxy)-2-(trifluoromethyl)phenoxy]phenyl]acetic acid;
2-[3-[4-(3,4-dihydro-2H-1,4-benzoxazin-6-yloxy)-2-(trifluoromethyl)phenoxy]phenyl]acetic acid;
2-[3-[4-([1,2,4]triazolo [4,3-a]pyridin-7-yloxy)-2-(trifluoromethyl)phenoxy] phenyl] acetic acid;
2-[3-[4-[1-(oxetan-3-yl)indol-6-yl]oxy-2-(trifluoromethyl)phenoxy]phenyl]acetic acid;
2-[3-[4-[1-(oxetan-3-yl)indazol-6-yl]oxy-2-(trifluoromethyl)phenoxy]phenyl]acetic acid;
2-[3-[4-[2-(oxetan-3-yl)indazol-6-yl]oxy-2-(trifluoromethyl)phenoxy]phenyl]acetic acid;
2-[3-[4-[1-(oxetan-3-yl)indazol-5-yl]oxy-2-(trifluoromethyl)phenoxy]phenyl]acetic acid;
2-[3-[4-[2-(oxetan-3-yl)indazol-5-yl]oxy-2-(trifluoromethyl)phenoxy]phenyl]acetic acid;
2-[3-[4-[3-(oxetan-3-yl)benzotriazol-5-yl]oxy-2-(trifluoromethyl) phenoxy]phenyl]acetic acid;
2-[3-[4-[3-(oxetan-3-yl)benzimidazol-5-yl]oxy-2-(trifluoromethyl)phenoxy] phenyl]acetic acid;
2-[3-[4-[2-methyl-3-(oxetan-3-yl)benzimidazol-5-yl]oxy-2-(trifluoromethyl) phenoxy] phenyl]acetic acid;
2-[3-[2-(difluoromethyl)-4-[2-methyl-3-(oxetan-3-yl)benzimidazol-5-yl]oxy-phenoxy] phenyl]acetic acid;
2-[3-[4-[1-(oxetan-3-yl)imidazo[4,5-b]pyridin-6-yl]oxy-2-(trifluoromethyl) phenoxy] phenyl]acetic acid;
2-[3-[4-[1-(oxetan-3-yl)pyrrolo[2,3-b]pyridin-6-yl]oxy-2-(trifluoromethyl) phenoxy] phenyl]acetic acid;
6-[4-[3-(carboxymethyl)phenoxy]-3-(trifluoromethyl)phenoxy]imidazo[1,5-a]pyridine-3-carboxylic acid;
2-[3-[4-[1-(carboxymethyl)indol-5-yl]oxy-2-(trifluoromethyl)phenoxy]phenyl]acetic acid;
2-[3-[4-[1-(carboxymethyl)indol-6-yl]oxy-2-(trifluoromethyl)phenoxy]phenyl]acetic acid;
3-[4-[4-(8-isoquinolyloxy)-2-(trifluoromethyl)phenoxy]phenyl]propanoic acid;
3-[3-[4-(2-methylindazol-5-yl)oxy-2-(trifluoromethyl)phenoxy]phenyl]propanoic acid;
2-[3-[[5-(8-isoquinolyloxy)-3-(trifluoromethyl)-2-pyridyl]oxy]phenyl]acetic acid;
3-[3-[4-imidazo[1,2-a]pyridin-5-yloxy-2-(trifluoromethyl)phenoxy]phenyl]propanoic acid;
2-[3-[2-cyclopropyl-4-(8-isoquinolyloxy)phenoxy]phenyl]acetic acid;
2-[3-[2-cyclopropyl-4-[2-methyl-3-(oxetan-3-yl)benzimidazol-5-yl]oxy-phenoxy] phenyl]acetic acid;
2-[3-[4-[(1-chloro-8-isoquinolyl)oxy]-2-(trifluoromethyl)phenoxy]phenyl]acetic acid;
2-[3-[4-[(1-methyl-8-isoquinolyl)oxy]-2-(trifluoromethyl)phenoxy]phenyl]acetic acid;
3-[3-[4-(8-isoquinolyloxy)-2-(trifluoromethyl)phenoxy]phenyl]propanoic acid;
3-[3-[4-(1,2,3,4-tetrahydroisoquinolin-8-yloxy)-2-(trifluoromethyl) phenoxy] phenyl] propanoic acid;
3-[3-[4-indan-1-yloxy-2-(trifluoromethyl)phenoxy]phenyl]propanoic acid;
3-[3-[4-(1H-indazol-5-yloxy)-2-(trifluoromethyl)phenoxy]phenyl]propanoic acid;
2-[3-[4-[(3-cyano-1H-indazol-5-yl)oxy]-2-(trifluoromethyl) phenoxy]phenyl]acetic acid;
5-[4-[3-(carboxymethyl)phenoxy]-3-(trifluoromethyl)phenoxy]-1H-indazole-3-carboxylic acid;
2-[3-[4-(1H-indazol-4-yloxy)-2-(trifluoromethyl)phenoxy]phenyl]acetic acid;
2-[3-[4-imidazo[1,5-a]pyridin-5-yloxy-2-(trifluoromethyl)phenoxy]phenyl]acetic acid;
2-[3-[4-imidazo[1,5-a]pyridin-8-yloxy-2-(trifluoromethyl) phenoxy]phenyl]acetic acid;
2-[3-[4-(3-methylimidazo[1,5-a]pyridin-5-yl)oxy-2-(trifluoromethyl)phenoxy]phenyl]acetic acid;
2-[3-[4-(3-methylimidazo[1,5-a]pyridin-8-yl)oxy-2-(trifluoromethyl)phenoxy]phenyl]acetic acid;
2-[3-[4-[3-(oxetan-3-yl)imidazo[1,5-a]pyridin-6-yl]oxy-2-(trifluoromethyl) phenoxy] phenyl] acetic acid;
2-[3-[4-(8-isoquinolyloxy)-2-(trifluoromethyl)phenoxy]phenyl]-N-methylsulfonyl-acetamide;
3-[3-[4-(8-isoquinolyloxy)-2-(trifluoromethyl)phenoxy]phenyl]propanamide;
2-[3-[4-(8-isoquinolyloxy)-2-(trifluoromethyl)phenoxy]phenyl]acetamide;
8-[4-[3-[2-(4H-1,2,4-triazol-3-yl)ethyl]phenoxy]-3-(trifluoromethyl)phenoxy]isoquinoline;
8-[4-[3-[2-(1H-tetrazol-5-yl)ethyl]phenoxy]-3-(trifluoromethyl)phenoxy]isoquinoline;
8-[4-[3-(1H-tetrazol-5-ylmethyl)phenoxy]-3-(trifluoromethyl) phenoxy]isoquinoline; and
2-[3-[4-(8-isoquinolyloxy)-3-(trifluoromethyl)phenoxy]phenyl]acetic acid;
or a pharmaceutically acceptable salt thereof.

8. The inhibitor of GPR91 for use according to any one of claims 1-6, wherein the inhibitor of GPR91 is selected from the group consisting of:
2-(2-(4'-((4-methylpiperazin-1-yl)methyl)-[1,1'-biphenyl]-3 - carboxamido)phenyl)acetic acid having a structure represented by 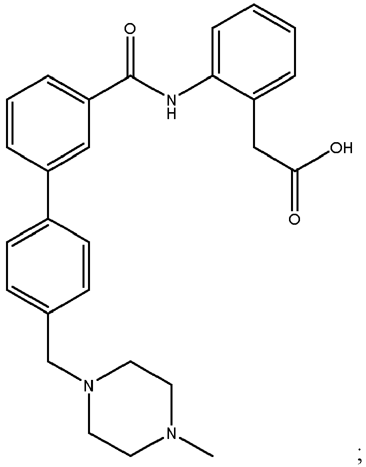
N-[{3-(3-trifluoromethyl-4-fluorophenyl)isoxazol-5-yl}methyl]-4-([1,8]naphthyridin-2-yl)butyramide) having a structure represented by 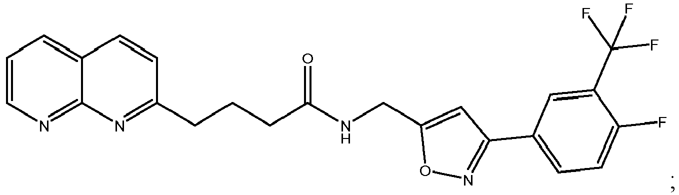 or
a pharmaceutically acceptable salt thereof.

9. The inhibitor of GPR91 for use according to any one of claims 1-6, wherein the use further comprises administration of an effective amount of an androgen receptor antagonist, JAK inhibitor, minoxidil, finasteride, dutasteride, antiandrogens, hair transplantation procedures, prostaglandin analogues, light therapy, platelet-rich plasma, or microneedling.

10. The inhibitor of GPR91 according to any one of Claims 19, wherein the subject has a decreased number of hair follicles in the anagen phase relative to a control subject prior to administering a therapeutically effective amount of a GPR91 inhibitor.

11. The inhibitor of GPR91 according to any one of Claims 1-10, wherein the subject is afflicted with, or is at risk of developing, alopecia from an underlying health disorder or genetic disposition.

12. Use of an inhibitor of GPR91 in the manufacture of a medicament for use in the treatment or prevention of a hair-loss related disorder or a symptom thereof in a subject in need thereof.

13. The use according to Claim 12, wherein the hair-loss related disorder is selected from alopecia areata, alopecia universalis, alopecia totalis, androgenetic alopecia, and telogen effluvium.

14. The use according to Claim 13, wherein the hair-loss related disorder is androgenetic alopecia.

15. An inhibitor of GPR91 for use in inducing, or promoting, hair growth in a human.

16. The inhibitor of GPR91 for use according to Claim 15 wherein inducing hair growth induces a transition from a telogen phase to an anagen phase of a hair growth cycle.

17. The inhibitor of GPR91 for use according to Claim 15 wherein the inhibitor of GPR91 increases a number of hair follicles present in the subject in the anagen phase.
